# EUROPÄISCHE PATENTSCHRIFT

(11) **EP 0 613 885 B1**
(45) Veröffentlichungstag und Bekanntmachung des Hinweises auf die Patenterteilung: **19.09.2001**
(21) Anmeldenummer: 94102324.4
(22) Anmeldetag: 16.02.1994
(51) Int. Cl.: C07D 207/38, C07F 9/572, A01N 43/36, A01N 57/08, A01N 57/24

(54) **Substituierte 1-H-3-Phenyl-5-cycloalkylpyrrolidin-2,4-dione, ihre Herstellung und ihre Verwendung als Schädlingsbekämpfungsmittel und Herbizide**
Subsituted 1-H-3-phenyl-5-cycloalkyl-pyrrolidin-2,4-diones, their preparation and their use as parasiticides and herbicides
Dérivées de la 1-H-3-phényl-5-cycloalkyl-pyrrolidine-2,4-dione substituée, leur préparation et leur utilisation comme parasiticides et herbicides

(30) Priorität: 01.03.1993 DE 4306257
(43) Veröffentlichungstag der Anmeldung: 07.09.1994
(73) Patentinhaber: BAYER AG, 51368 Leverkusen (DE)
(72) Erfinder: Fischer, Reiner, Dr., D-40789 Monheim (DE); Bretschneider, Thomas, Dr., D-53797 Lohmar (DE); Krüger, Bernd-Wieland, Dr., D-51467 Bergisch Gladbach (DE); Santel, Hans-Joachim, Dr., D-51371 Leverkusen (DE); Dollinger, Markus, Dr., D-51381 Leverkusen (DE); Turberg, Andreas, Dr., D-40699 Erkrath (DE); Wachendorff-Neumann, Ulricke, Dr., D-40789 Monnheim (DE)

(56) Entgegenhaltungen:
- EP-A- 0 456 063
- EP-A- 0 521 334
- EP-A- 0 595 130

## Beschreibung

Die Erfindung betrifft neue 1-H-3-Phenyl-5-cycloalkylpyrrolidin-2,4-dione, mehrere Verfahren zu ihrer Herstellung und ihre Verwendung als Schädlingsbekämpfungsmittel (insbesondere als Insektizide und Akarizide) und als Herbizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985 1095) synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A 355 599) und (EP 415 211), substituierte bicyclische 3-Aryl-pyrrolidin-2,4-dion-Derivate (EP 501 129) sowie substituierte mono-cyclische 3-Aryl-pyrrolidin-2,4-dion-Derivate (EP-A 377 893), (EP 442 077) und (EP 497 127).

Weiterhin bekannt sind polycyclische 3-Arylpyrrolidin-2,4-dion-Derivate (EP 442 073) sowie 1-H-3-Arylpyrrolidin-dion-Derivate (EP 456 063) und (EP 521 334).

Es wurden nun neue 1-H-3-Phenyl-5-cycloalkylpyrrolidin-2,4-dione der Formel (I) 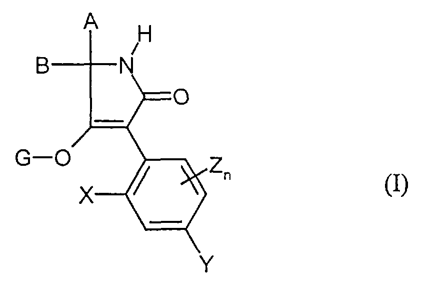
- A: für C₃-C₆-Cycloalkyl steht,
- B: für Methyl, Ethyl, Propyl, i-Propyl, Butyl, i-Butyl, s-Butyl oder t-Butyl steht,
- X: für Methyl, Ethyl, Propyl, i-Propyl, Fluor, Chlor oder Brom, steht,
- Y: für Methyl, Ethyl, Propyl, i-Propyl, Butyl, i-Butyl, tert.-Butyl, Fluor, Chlor oder Brom steht,
- Z: für Methyl, Ethyl, Propyl, i-Propyl, Butyl, i-Butyl oder tert.-Butyl, steht,
- n: für 0 oder 1 steht,
- G: für die Gruppen 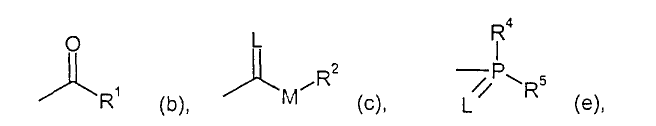 oder 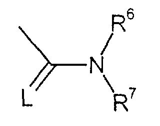 (g), steht, in welchen
L und M für Sauerstoff und/oder Schwefel stehen,
- R¹: für C₁-C₁₄-Alkyl steht,
- R²: für C₁-C₁₄-Alkyl steht,
- R⁴ und R⁵ unabhängig voneinander: für C₁-C₄-Alkyl oder C₁-C₄-Alkylthio stehen,
- R⁶ und R⁷: unabhängig voneinander für C₁-C₄-Alkyl oder zusammen mit dem N-Atom, an das sie gebunden sind, für einen gegebenenfalls durch Sauerstoff oder Schwefel unterbrochenen C₄-C₆-Alkylenring stehen.

Unter Einbeziehung der verschiedenen Bedeutungen (b), (c), (e) und (g) der Gruppe G der allgemeinen Formel (I) ergeben sich folgende hauptsächlichen Strukturen: 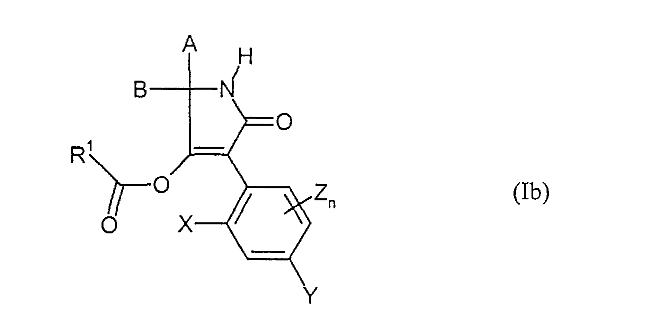 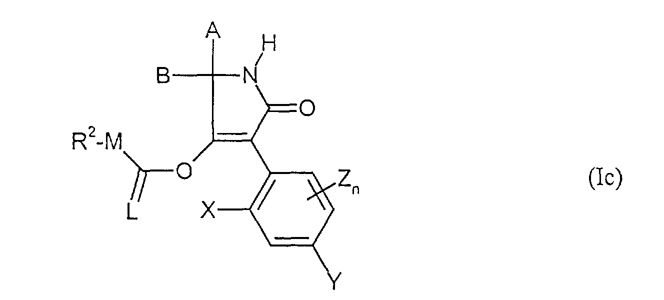 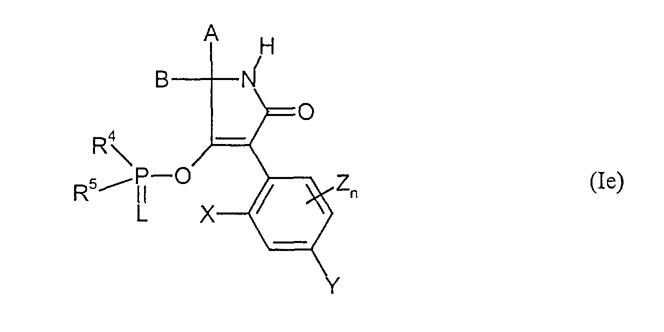 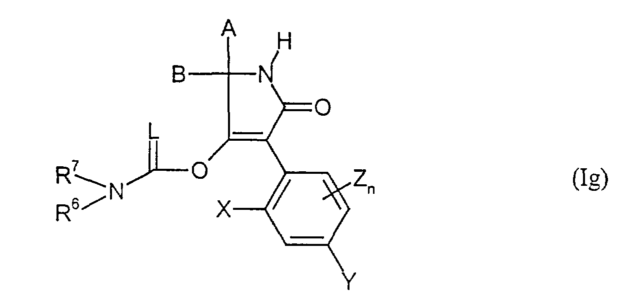 worin
A, B, L, M, X, Y, Zₙ, R¹, R², R⁴, R⁵, R⁶ und R⁷ die oben angegebenen Bedeutungen besitzen.

Aufgrund eines oder mehrerer Chiralitätszentren, fallen die Verbindungen der Formeln Ib, Ic, Ie und If im allgemeinen als Stereoisomerengemisch an, die gegebenenfalls in üblicher Art und Weise getrennt werden können. Sie können sowohl in Form ihrer Diastereomerengemische als auch als reine Diastereomere oder Enantiomere verwendet werden. Im folgenden wird der Einfachheit halber stets von Verbindungen der Formeln Ib, Ic, Ie und If gesprochen, obwohl sowohl die reinen Verbindungen, als auch die Gemische mit unterschiedlichen Anteilen an isomeren, enantiomeren und stereomeren Verbindungen gemeint sind.

Weiterhin wurde gefunden, daß man die neuen substituierten 1-H-3-Phenyl-5-cycloalkylpyrrolidin-2,4-dione der Formel (I) nach einem der im folgenden beschriebenen Verfahren erhält.
(B) Man erhält Verbindungen der Formel (Ib) 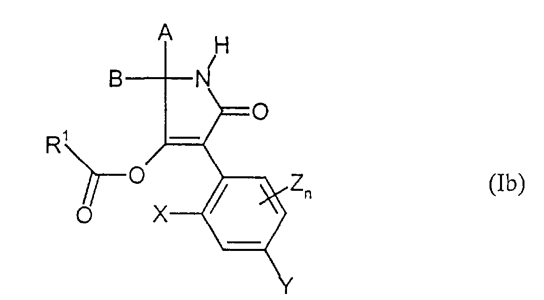 in welcher
   A, B, X, Y, Z, R¹ und n die oben angegebene Bedeutung haben,
   wenn man Verbindungen der Formel (Ia), 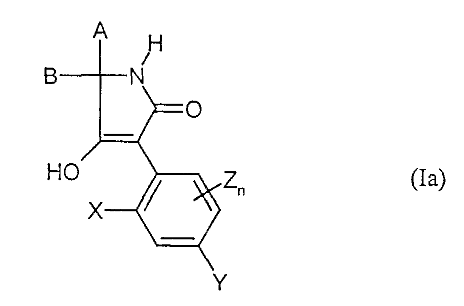 in welcher
   A, B, X, Y, Z und n die oben angegebene Bedeutung haben,
   α) mit Säurehalogeniden der allgemeinen Formel (III) 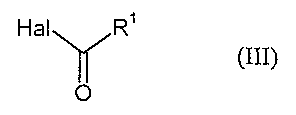 in welcher
      - R¹: die oben angegebene Bedeutung hat und
      - Hal: für Halogen, insbesondere Chlor und Brom steht,
      gegebenenfalls in Gegenwart eines Verdünnungsmittels und gegebenenfalls in Gegenwart eines Säurebindemittels umsetzt
      oder
   β) mit Carbonsäureanhydriden der allgemeinen Formel (IV)

      R¹-CO-O-CO-R¹ (IV)

      in welcher
      - R¹: die oben angegebene Bedeutung hat,
      gegebenenfalls in Gegenwart eines Verdünnungsmittels und gegebenenfalls in Gegenwart eines Säurebindemittels, umsetzt;
   oder
(C) man erhält Verbindungen der Formel (Ic-1) 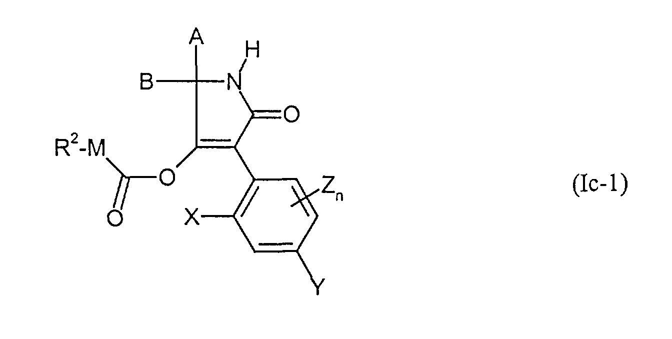 in welcher
   - A, B, X, Y, Z, R² und n: die oben angegebene Bedeutung haben,
   und
   - M: für Sauerstoff oder Schwefel steht,
   wenn man Verbindungen der Formel (Ia)
   in welcher
   - A, B, X, Y, Z und n: die oben angegebene Bedeutung haben,
   mit Chlorameisensäureester oder Chlorameisensäurethiolester der allgemeinen Formel (V)

   R²-M-CO-Cl (V)

   in welcher
   R² und M die oben angegebene Bedeutung haben,
   gegebenenfalls in Gegenwart eines Verdünnungsmittels und gegebenenfalls in Gegenwart eines Säurebindemittels umsetzt;
   oder
(D) man erhält Verbindungen der Formel (Ic-2) 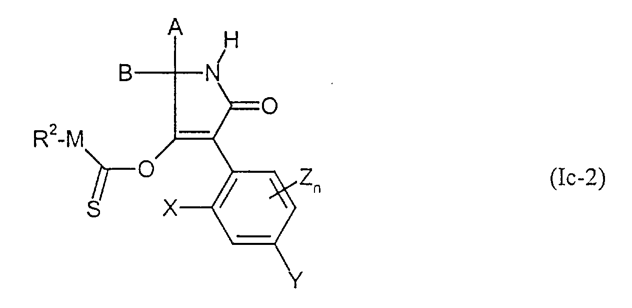 in welcher
   - A, B, R², X, Y, Z und n: die oben angegebene Bedeutung haben
   und
   - M: für Sauerstoff oder Schwefel steht,
   wenn man Verbindungen der Formel (Ia) 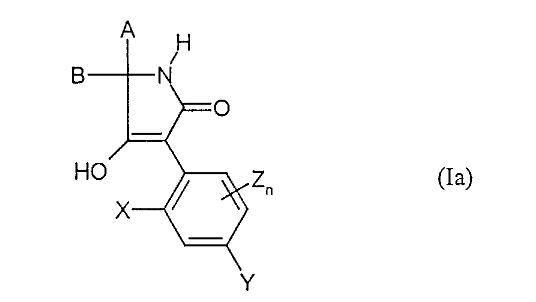 in welcher
   - A, B, X, Y, Z und n: die oben angegebene Bedeutung haben,

   α) mit Chlormonothioameisensäureestern oder Chlordithioameisensäureestern der allgemeinen Formel (VI) 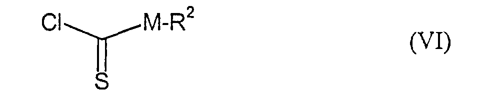 in welcher
      - M und R²: die oben angegebene Bedeutung haben,
      gegebenenfalls in Gegenwart eines Verdünnungsmittels und gegebenenfalls in Gegenwart eines Säurebindemittels umsetzt,
      oder
   β) mit Schwefelkohlenstoff und anschließend mit Alkylhalogeniden der allgemeinen Formel (VII)

      R²-Hal (VII)

      in welcher
      - R²: die oben angegebene Bedeutung hat
      und
      - Hal: für Chlor, Brom, Iod steht,
      gegebenenfalls in Gegenwart eines Verdünnungsmittels umsetzt;
   oder
(F) man erhält Verbindungen der Formel (Ie) 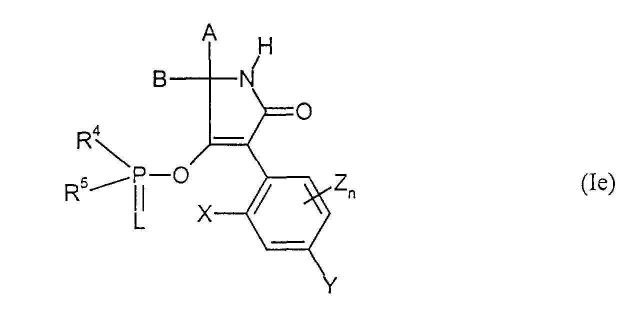 in welcher
   A, B, L, X, Y, Z, R⁴, R⁵ und n die oben angegebene Bedeutung haben,
   wenn man
   1-H-3-Phenyl-5-cycloalkyl pyrrolidin-2,4-dione der Formel (Ia) bzw. deren Enole 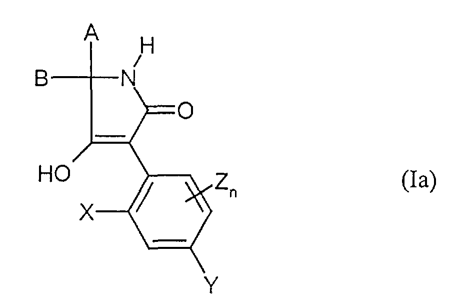 in welcher
   A, B, X, Y, Z und n die oben angegebene Bedeutung haben,
   mit Phosphorverbindungen der allgemeinen Formel (IX) 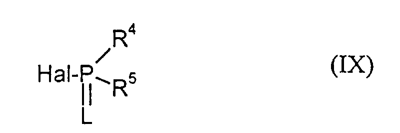 in welcher
   - L, R⁴ und R⁵: die oben angegebene Bedeutung haben
   und
   - Hal: für Halogen, insbesondere Chlor oder Brom steht,
   gegebenenfalls in Gegenwart eines Verdünnungsmittels und gegebenenfalls in Gegenwart eines Säurebindemittels umsetzt;
   oder
(H) Ferner wurde gefunden, daß man Verbindungen der Formel (I g) 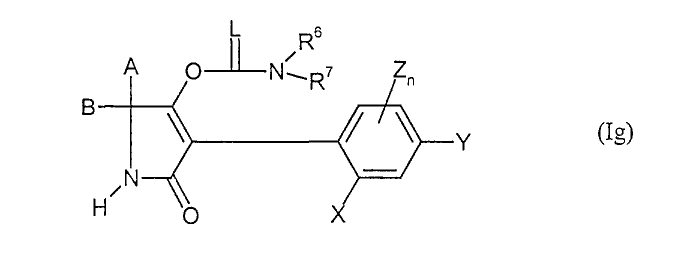 in welcher
   A, B, L, X, Y, Z, R⁶, R⁷ und n die oben angegebene Bedeutung haben,
   erhält, wenn man Verbindungen der Formel (I a) 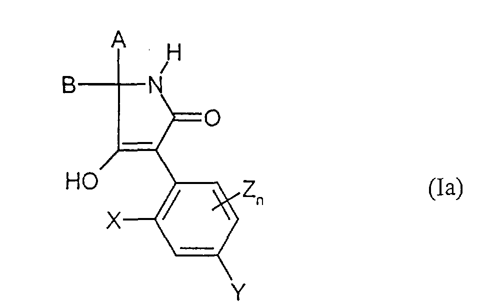 in welcher
   A, B, X, Y, Z und n die oben angegebene Bedeutung haben,
   α) mit Verbindungen der allgemeinen Formel (XII)

      R⁶-N=C=L (XII)

      in welcher
      - L und R⁶: die oben angegebene Bedeutung haben
      gegebenenfalls in Gegenwart eines Verdünnungsmittels und gegebenenfalls in Gegenwart eines Katalysators
      oder
   β) mit Carbamidsäurechloriden oder Thiocarbamidsäurechloriden der allgemeinen Formel (XIII) 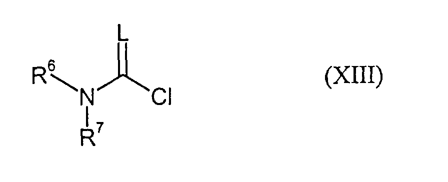 in welcher
      - L, R⁶ und R⁷: die oben angegebene Bedeutung haben
      gegebenenfalls in Gegenwart eines Verdünnungsmittels und gegebenenfalls in Gegenwart eines Säurebindemittels,
   umsetzt.

Weiterhin wurde gefunden, daß sich die neuen 1-H-3-Phenyl-5-cycloalkylpyrrolidin-2,4-dione der Formel (I) durch hervorragende insektizide, akarizide und herbizide Wirkungen auszeichnen.

Im einzelnen seien außer den bei den Herstellungsbeispielen genannten Verbindungen die folgenden Verbindungen der Formel (Ib) genannt:

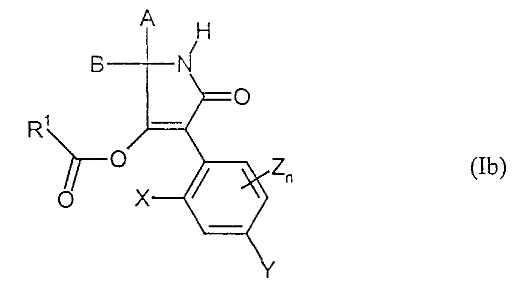

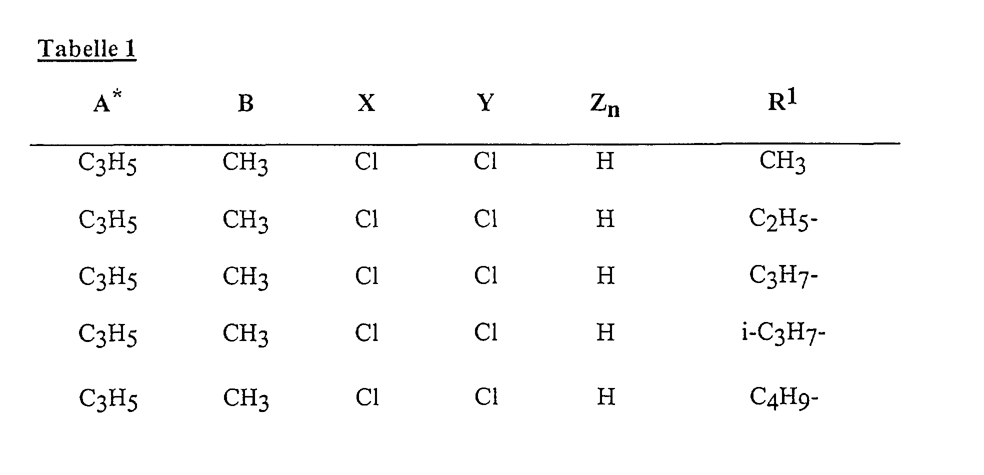

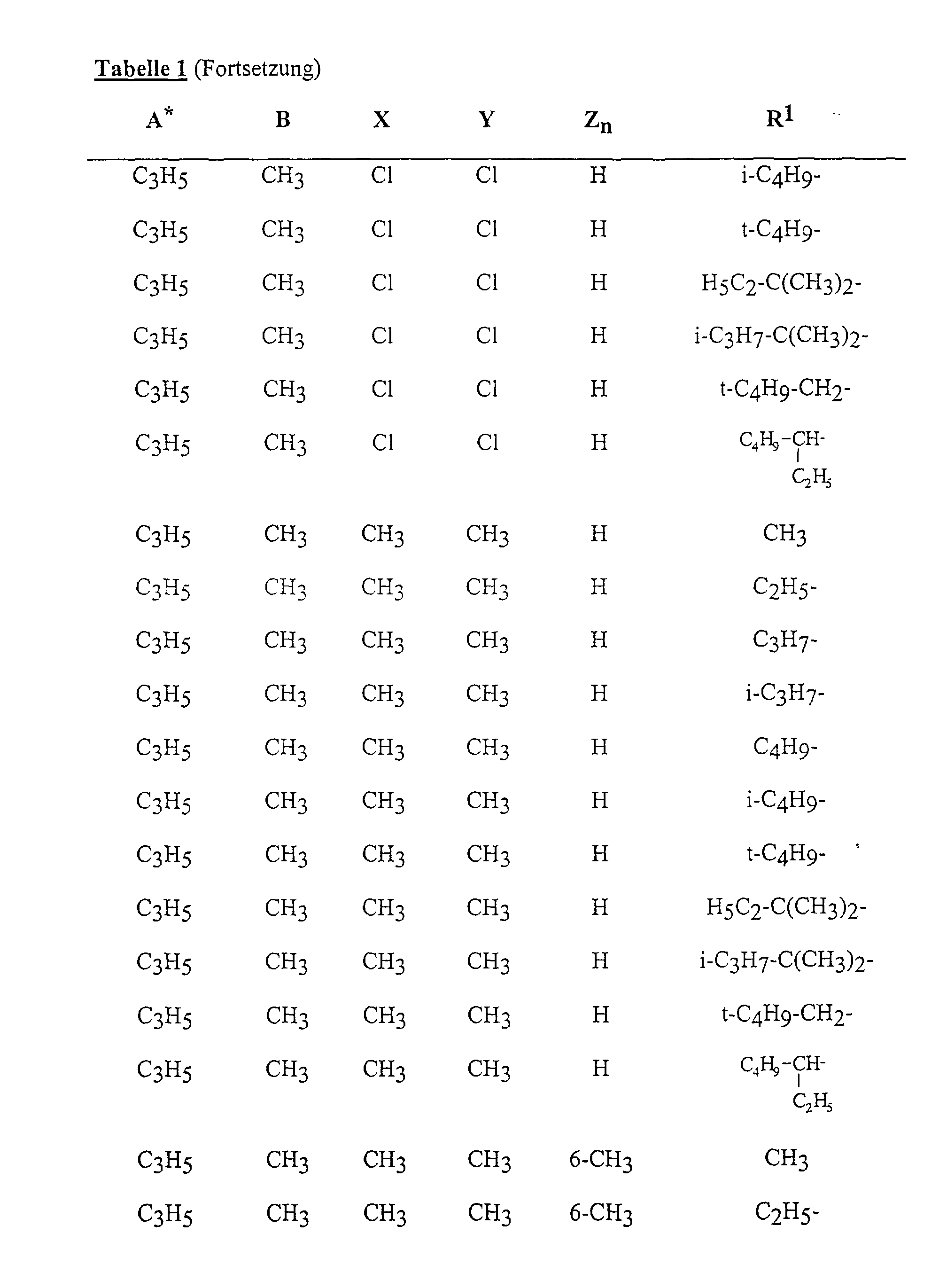

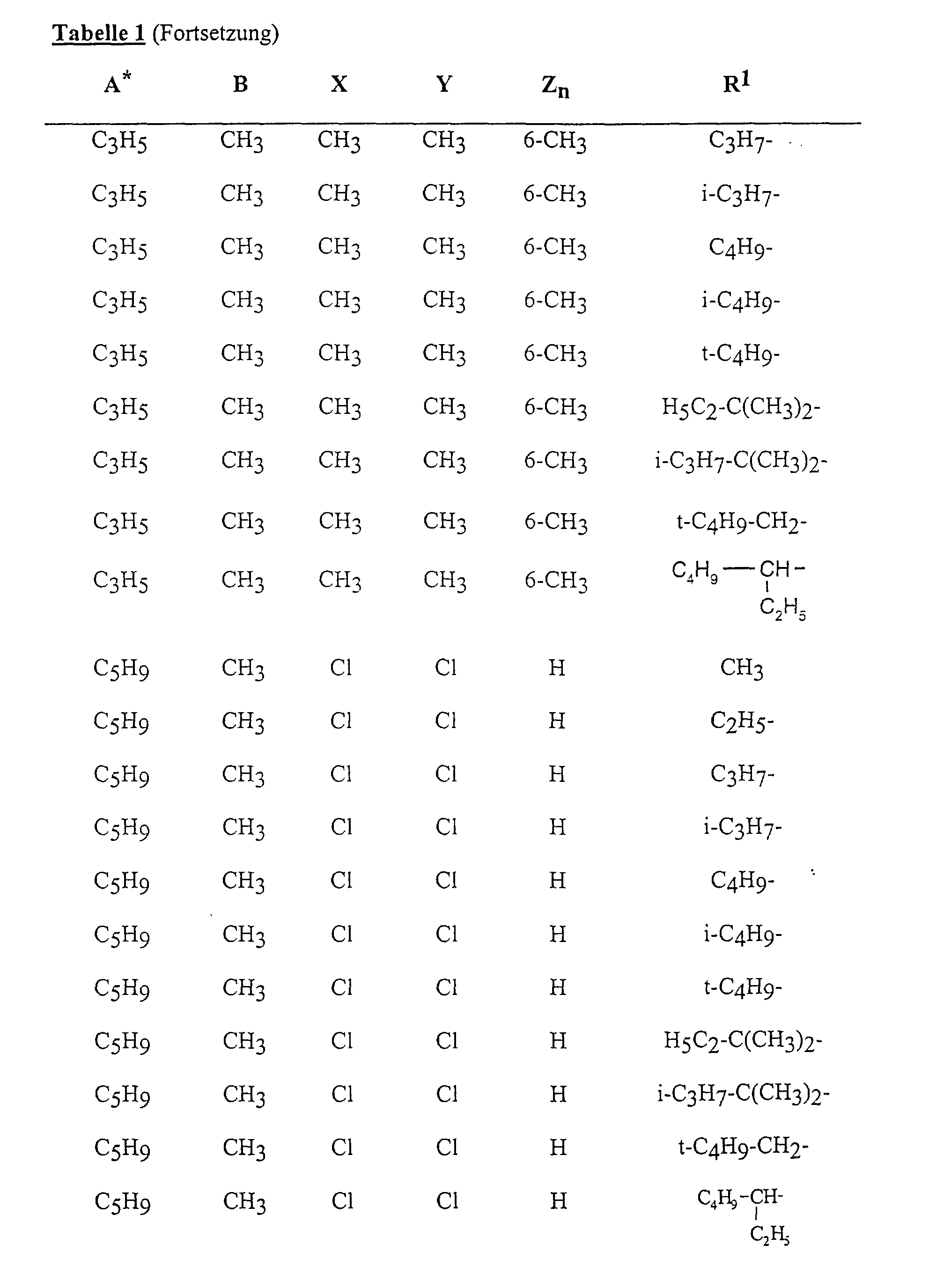

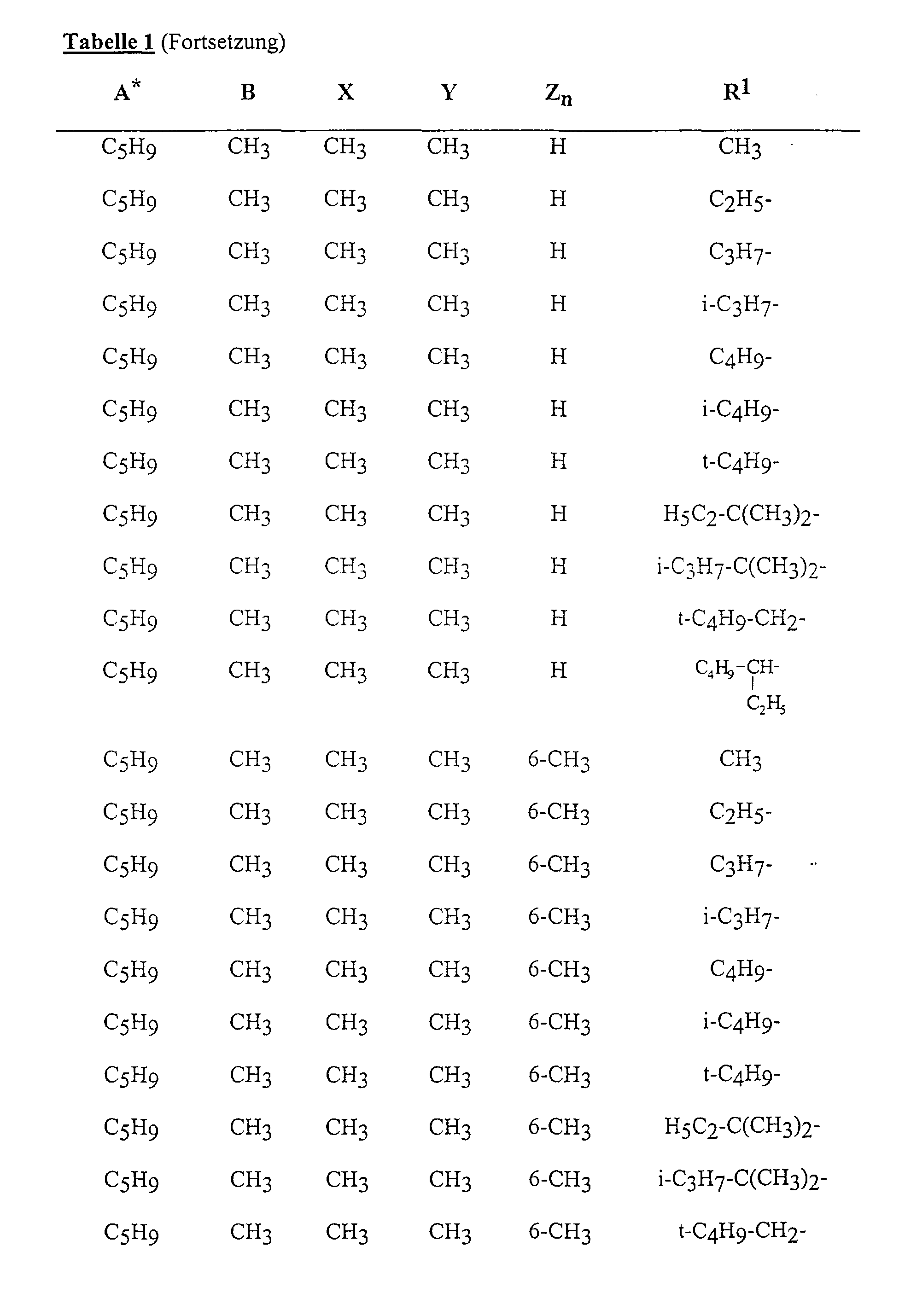

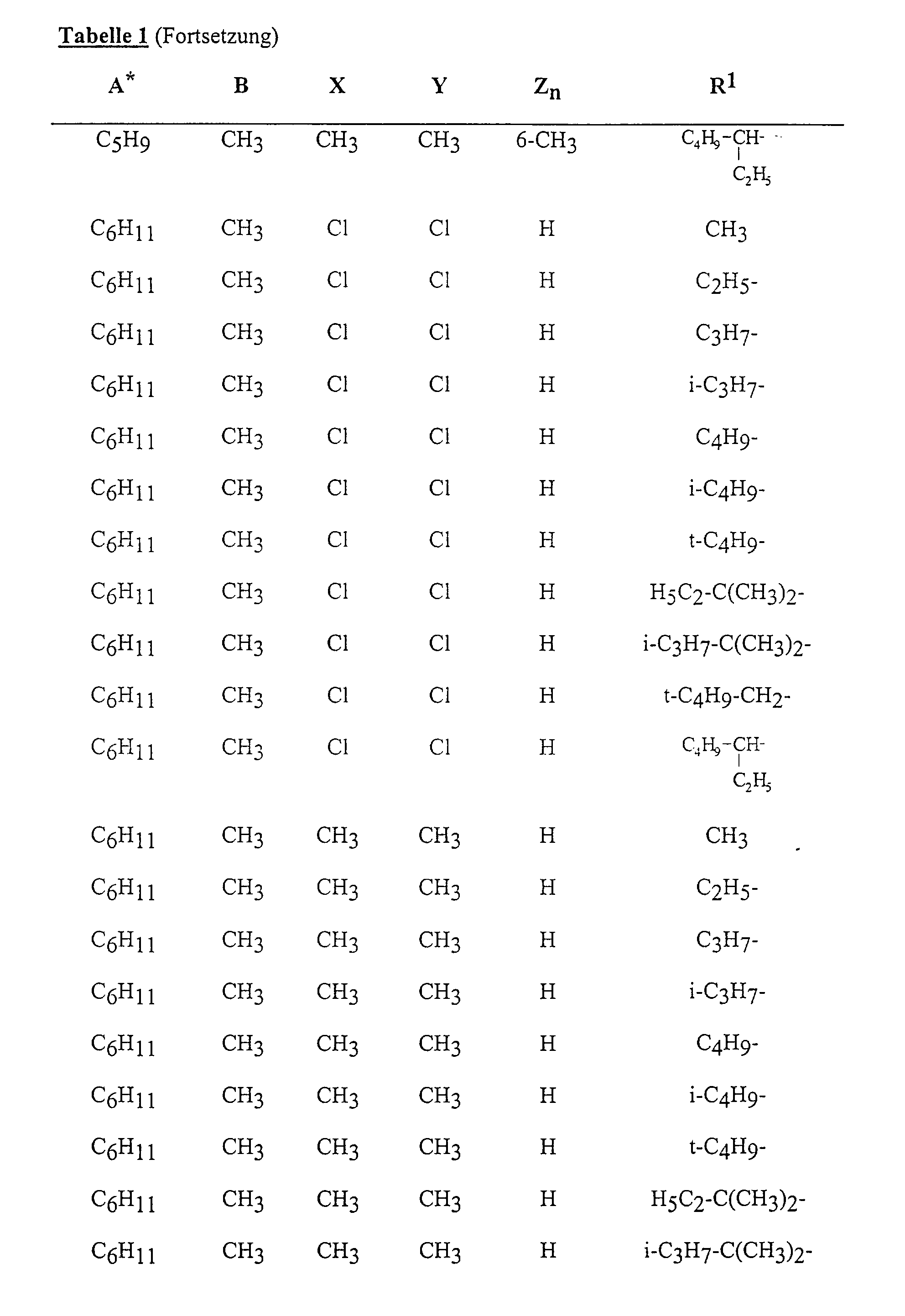

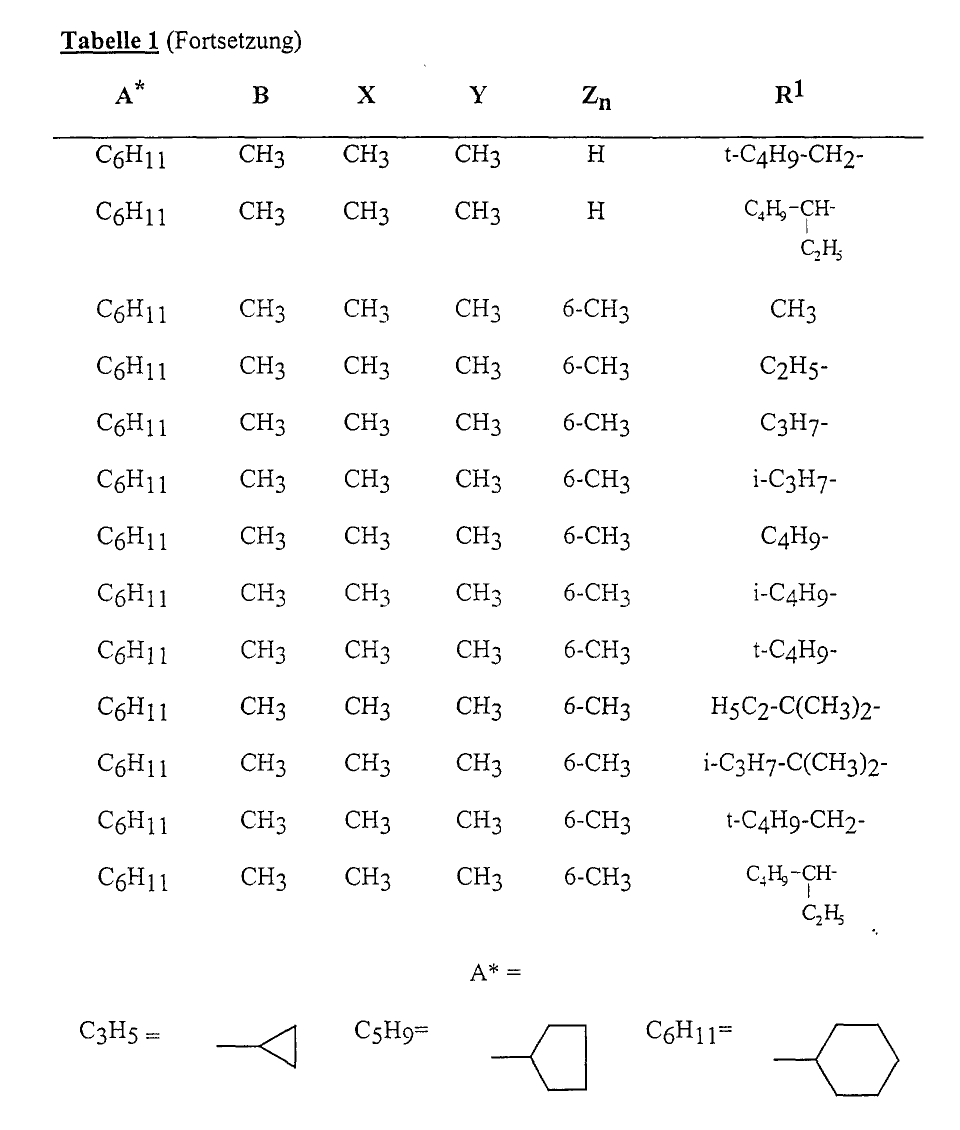

Im einzelnen seien außer den bei den Herstellungsbeispielen genannten Verbindungen die folgenden Verbindungen der Formel (Ic) genannt:

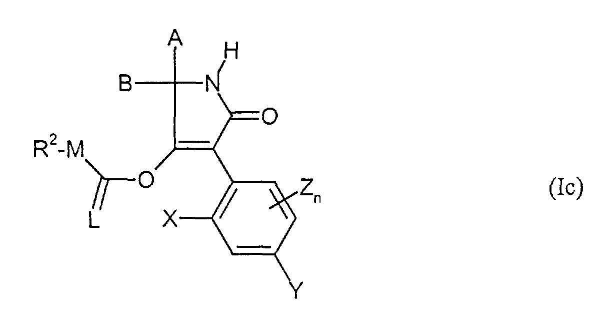

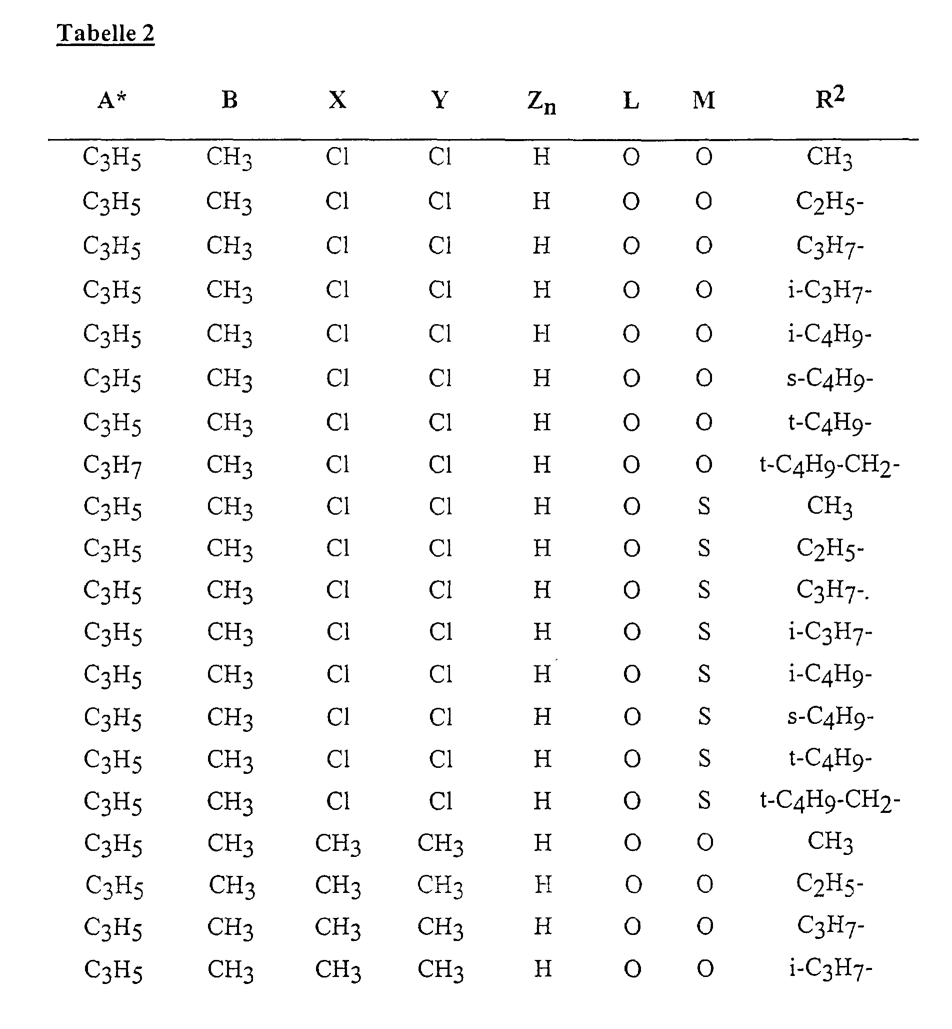

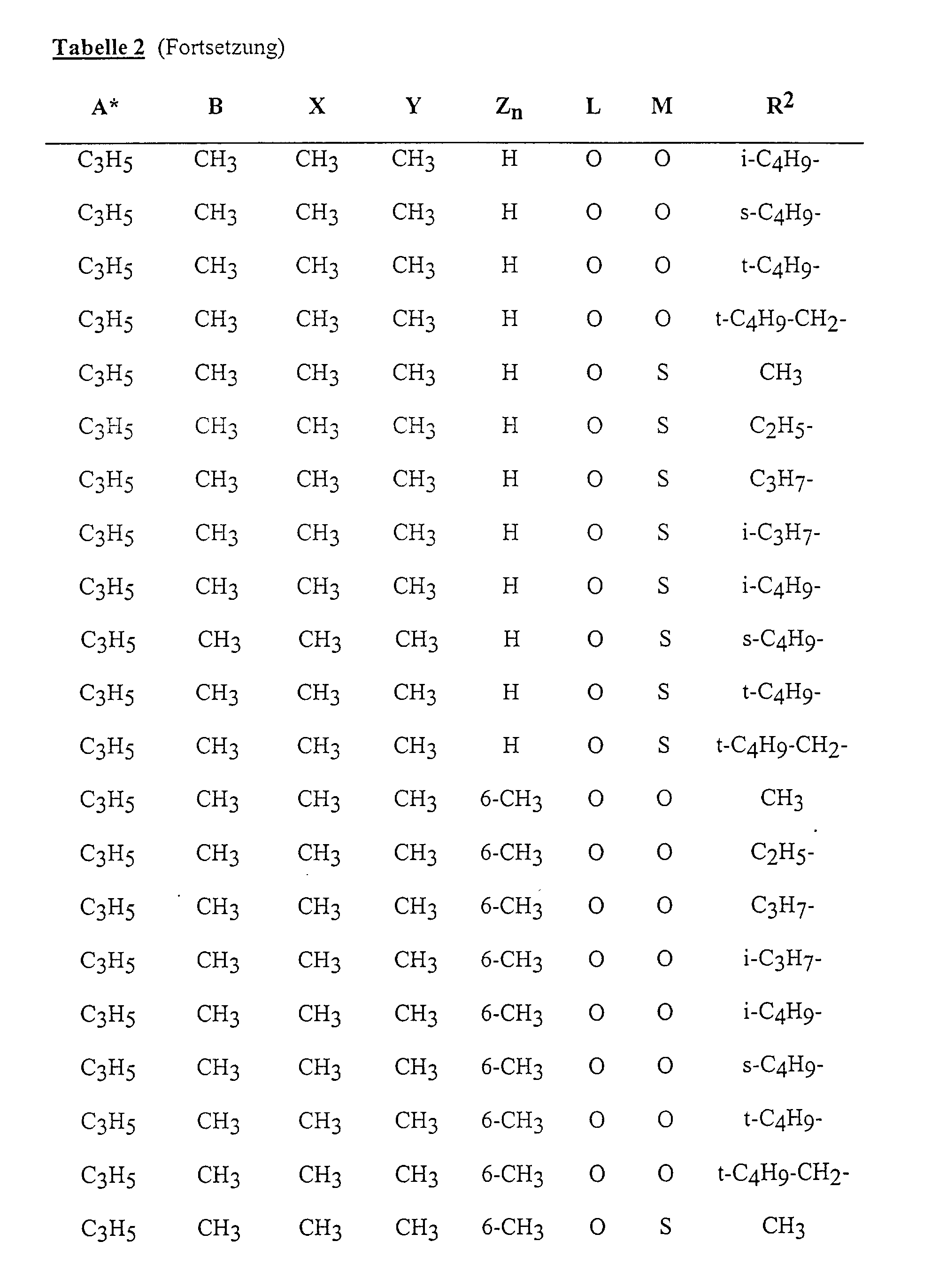

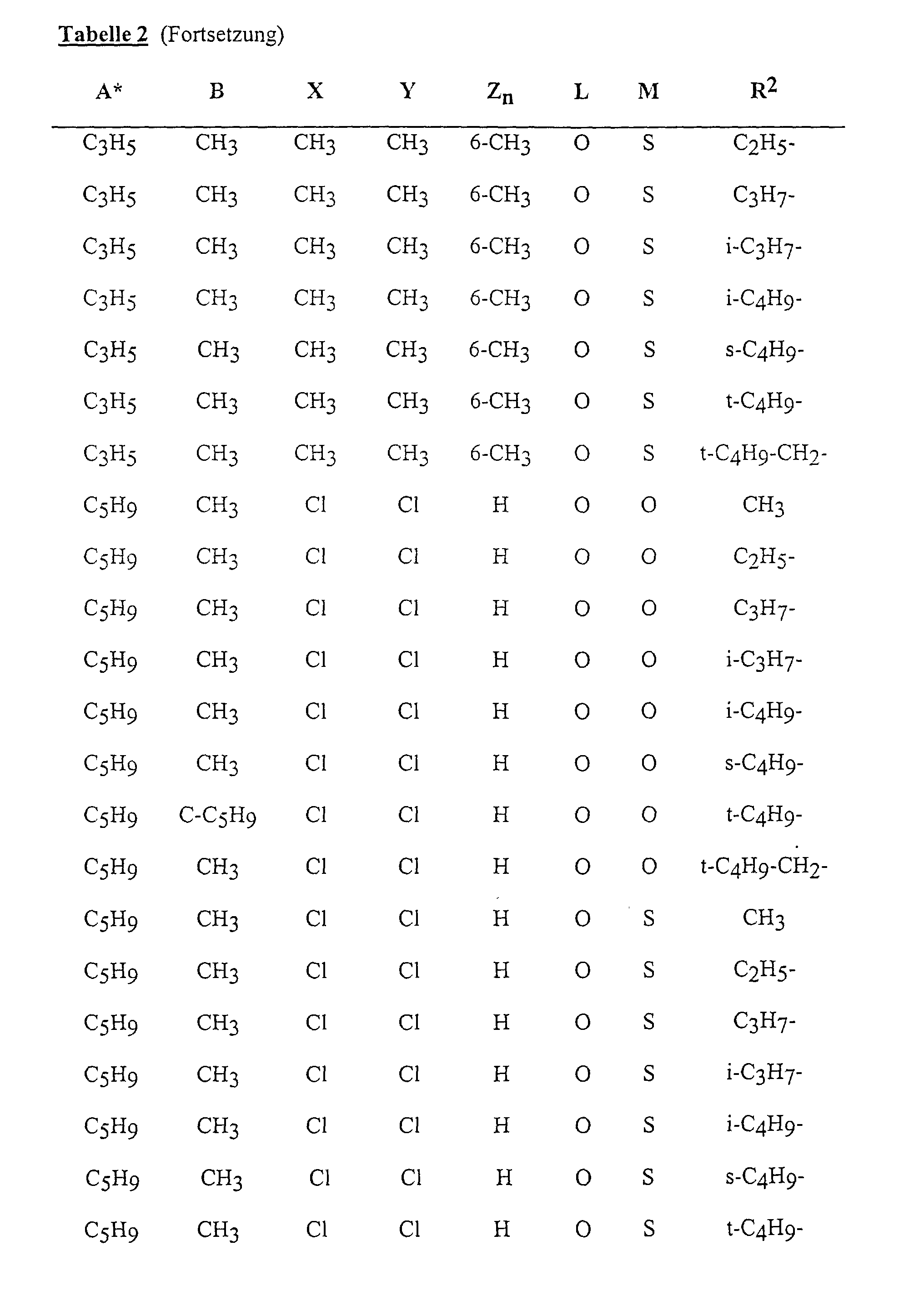

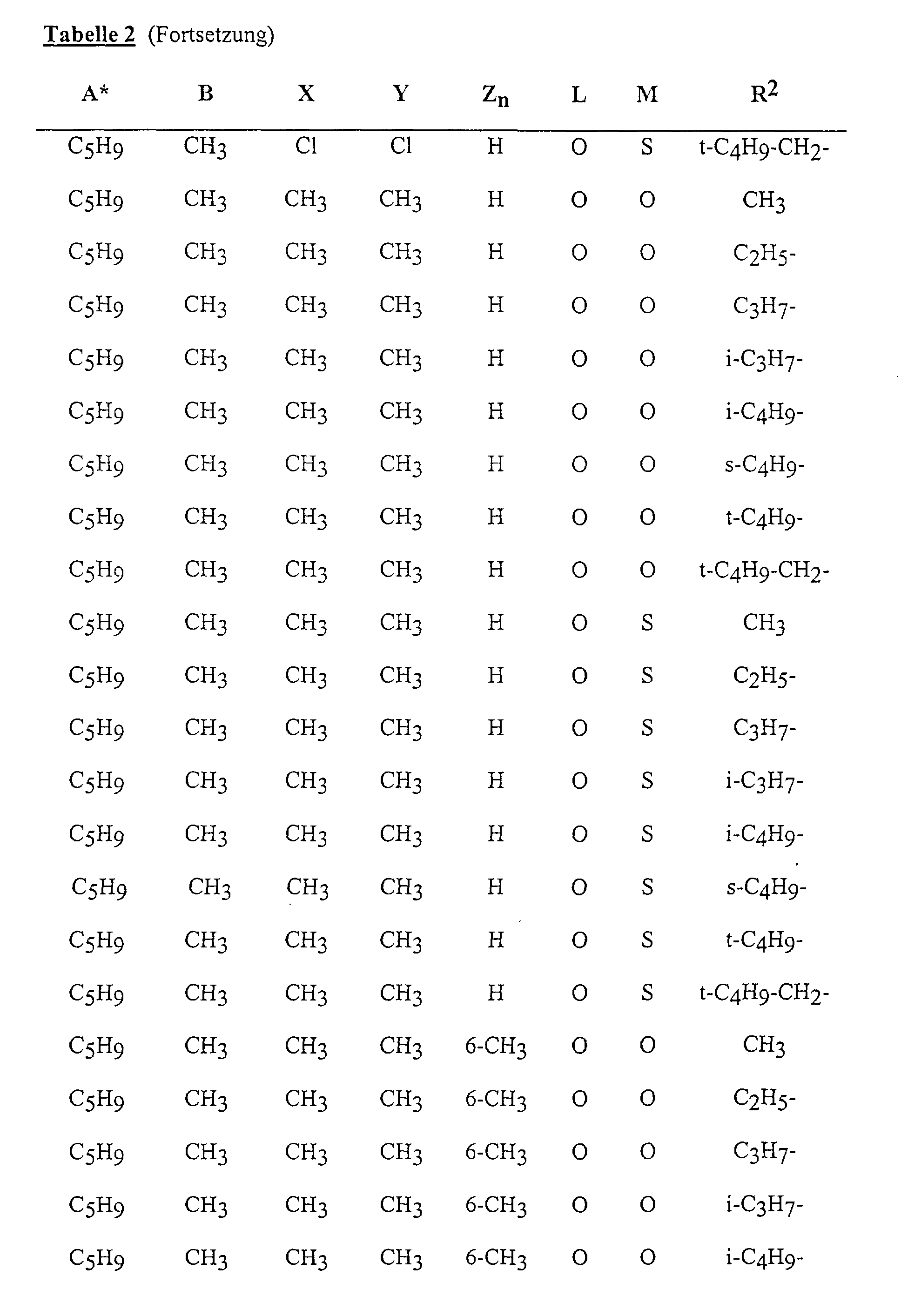

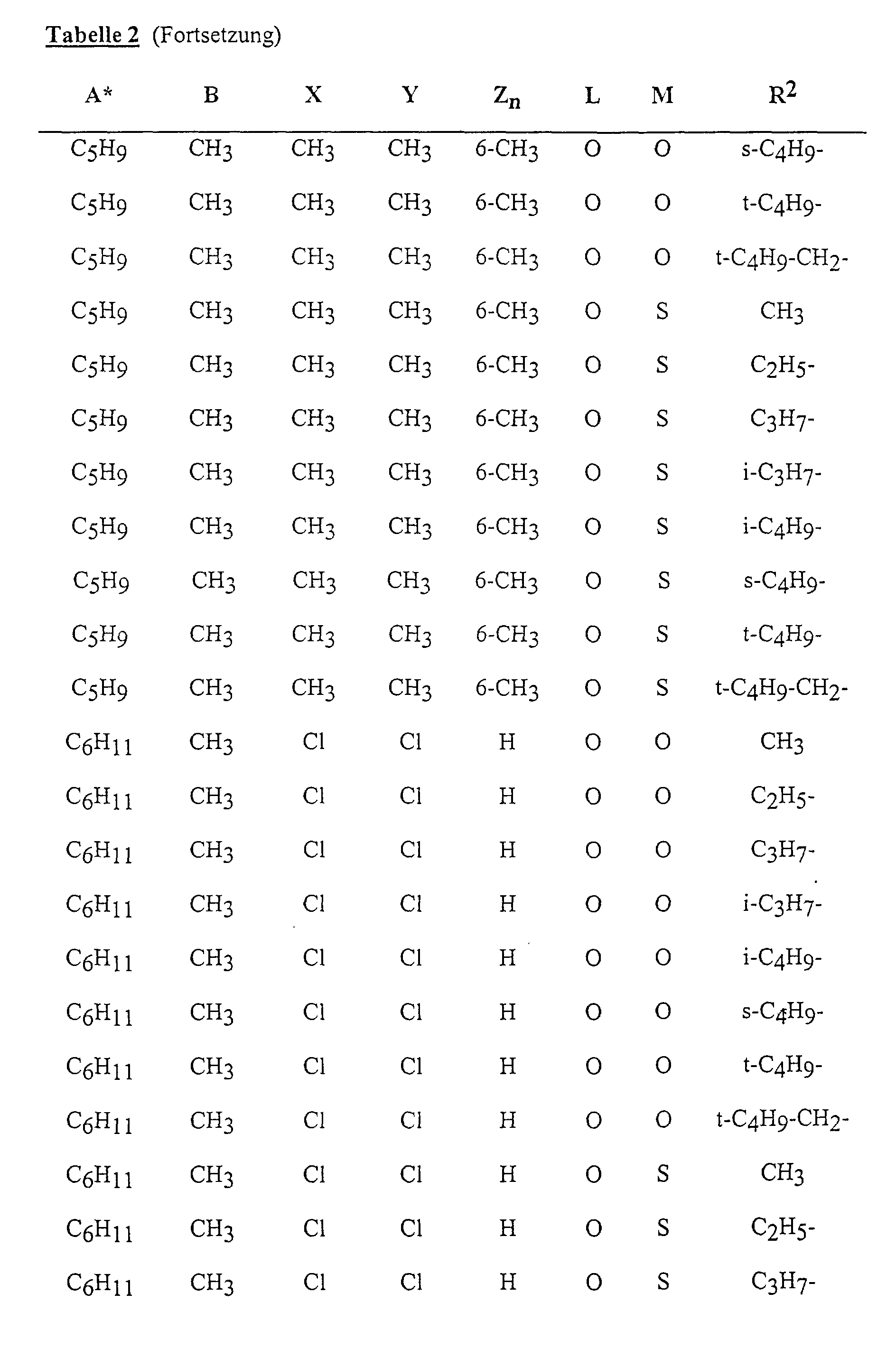

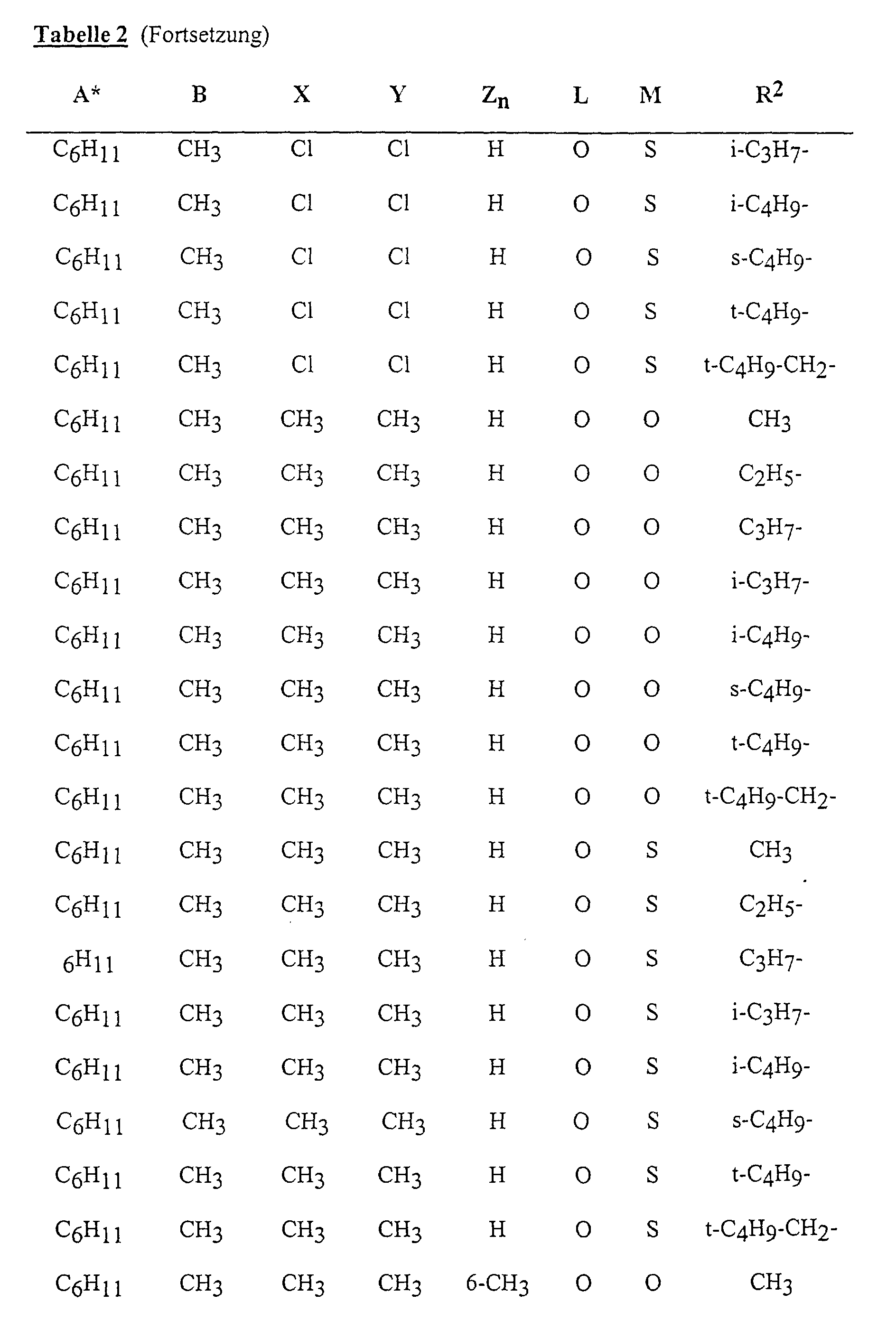

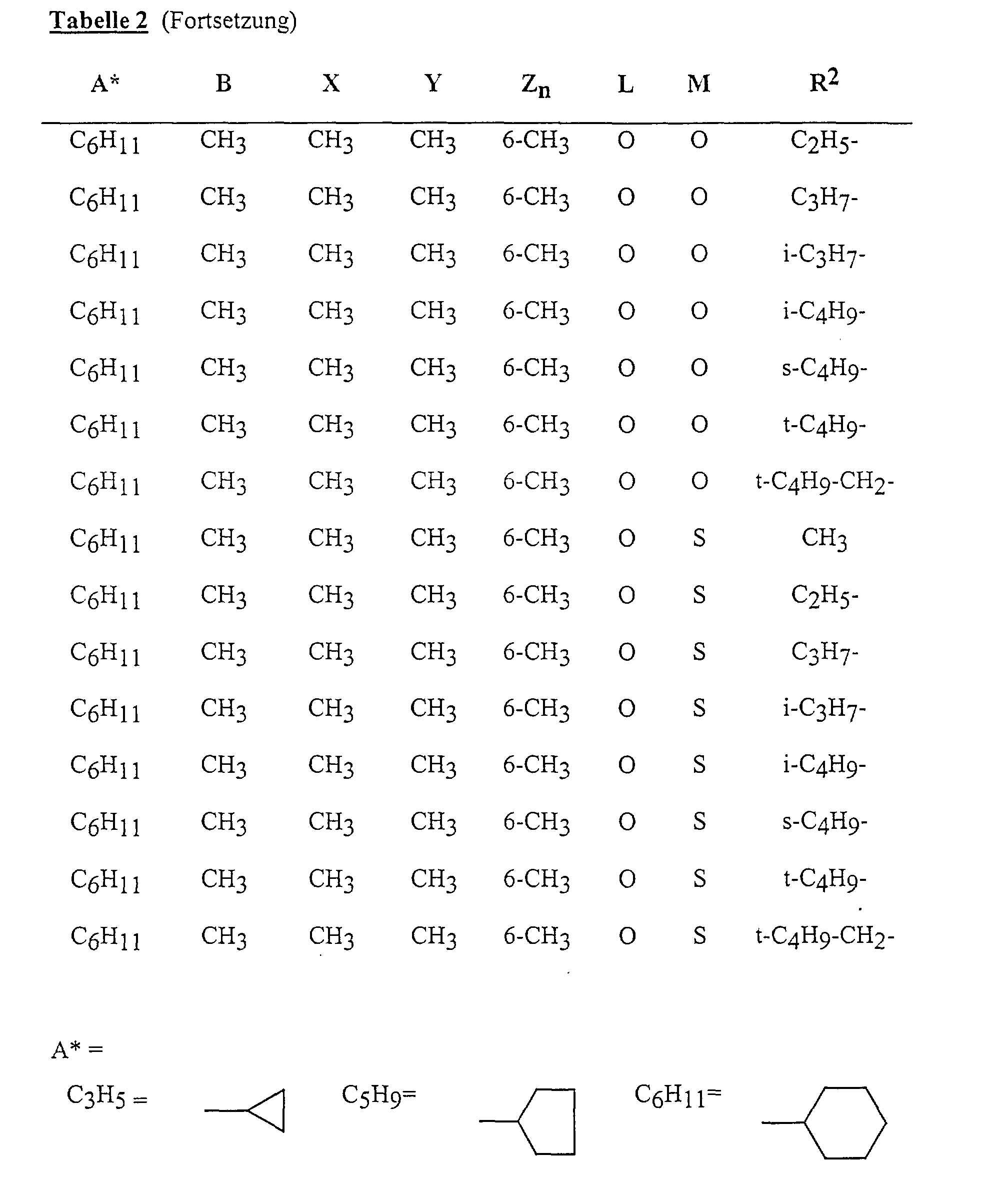

Im einzelnen seien außer den bei den Herstellungsbeispielen genannten Verbindungen die folgenden Verbindungen der Formel (Ie) bekannt:

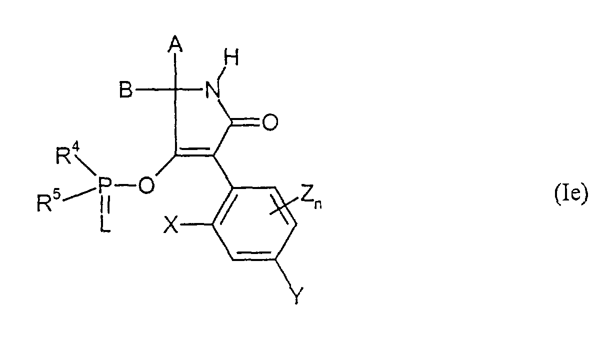

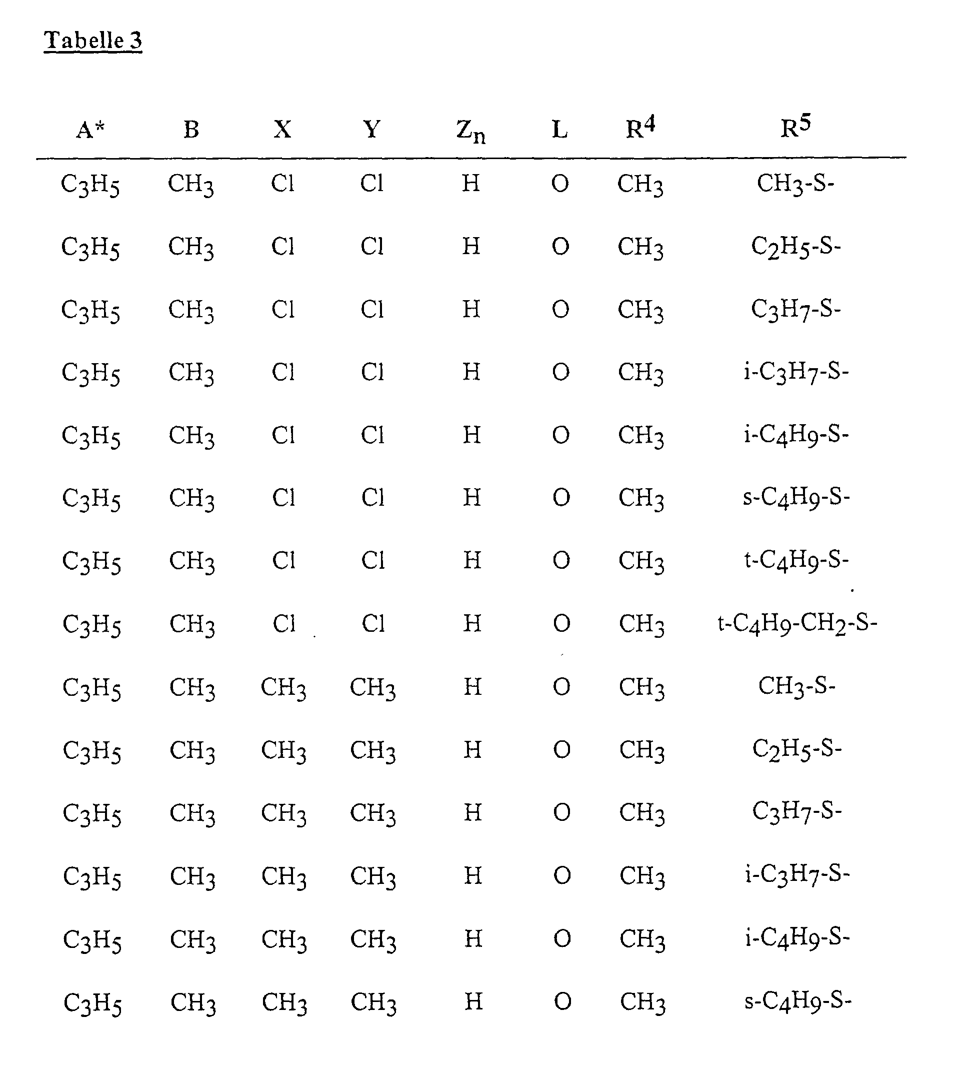

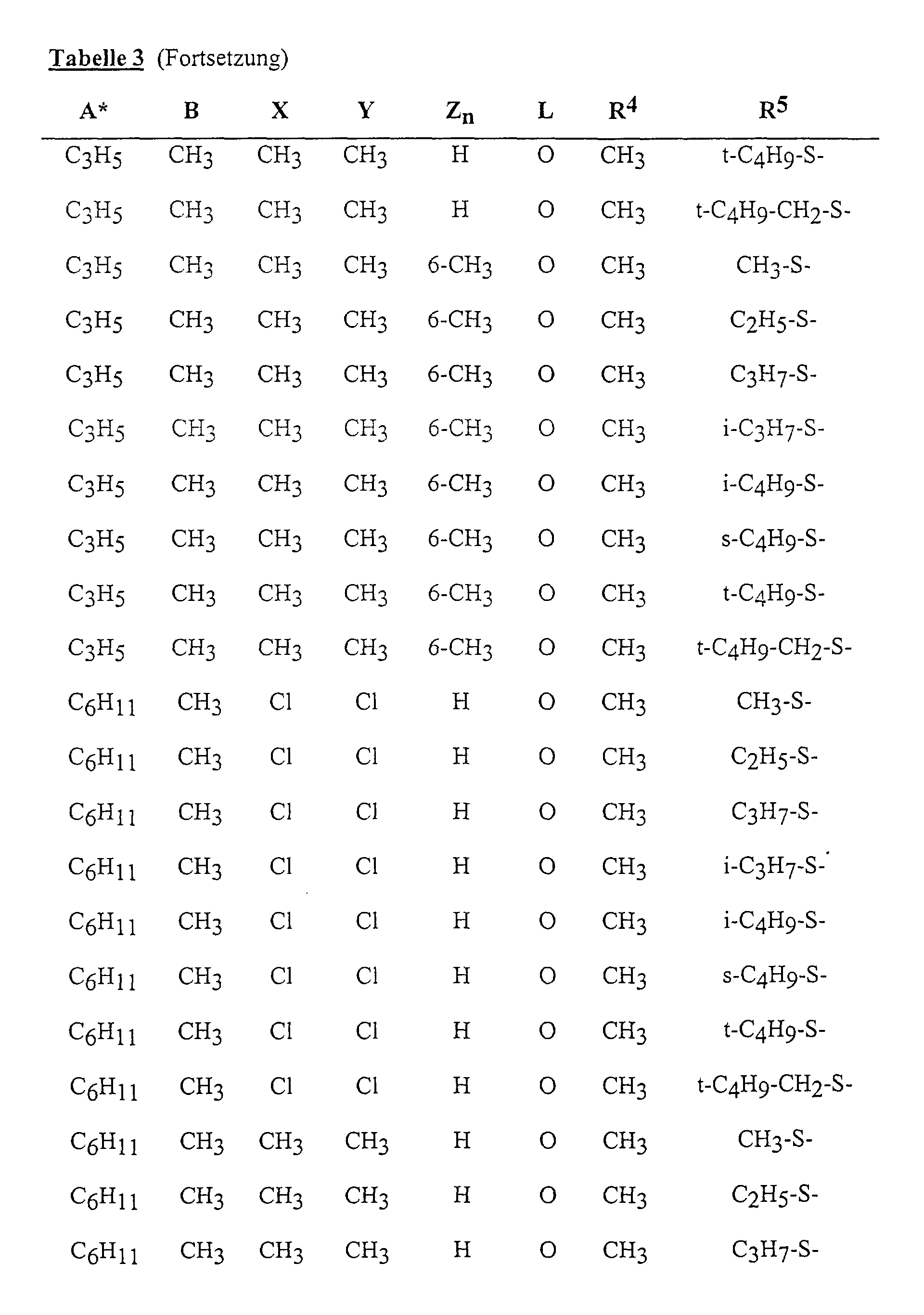

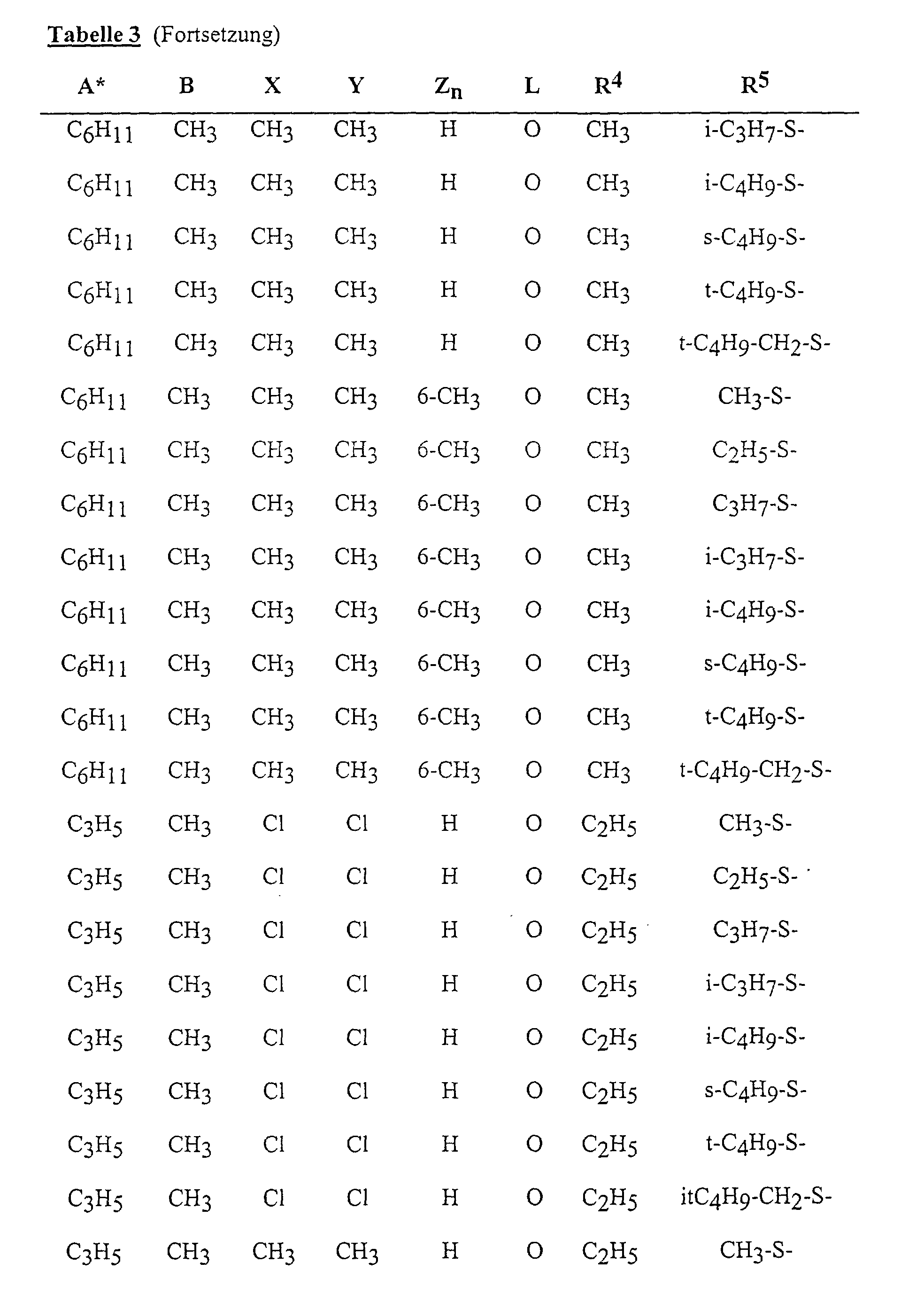

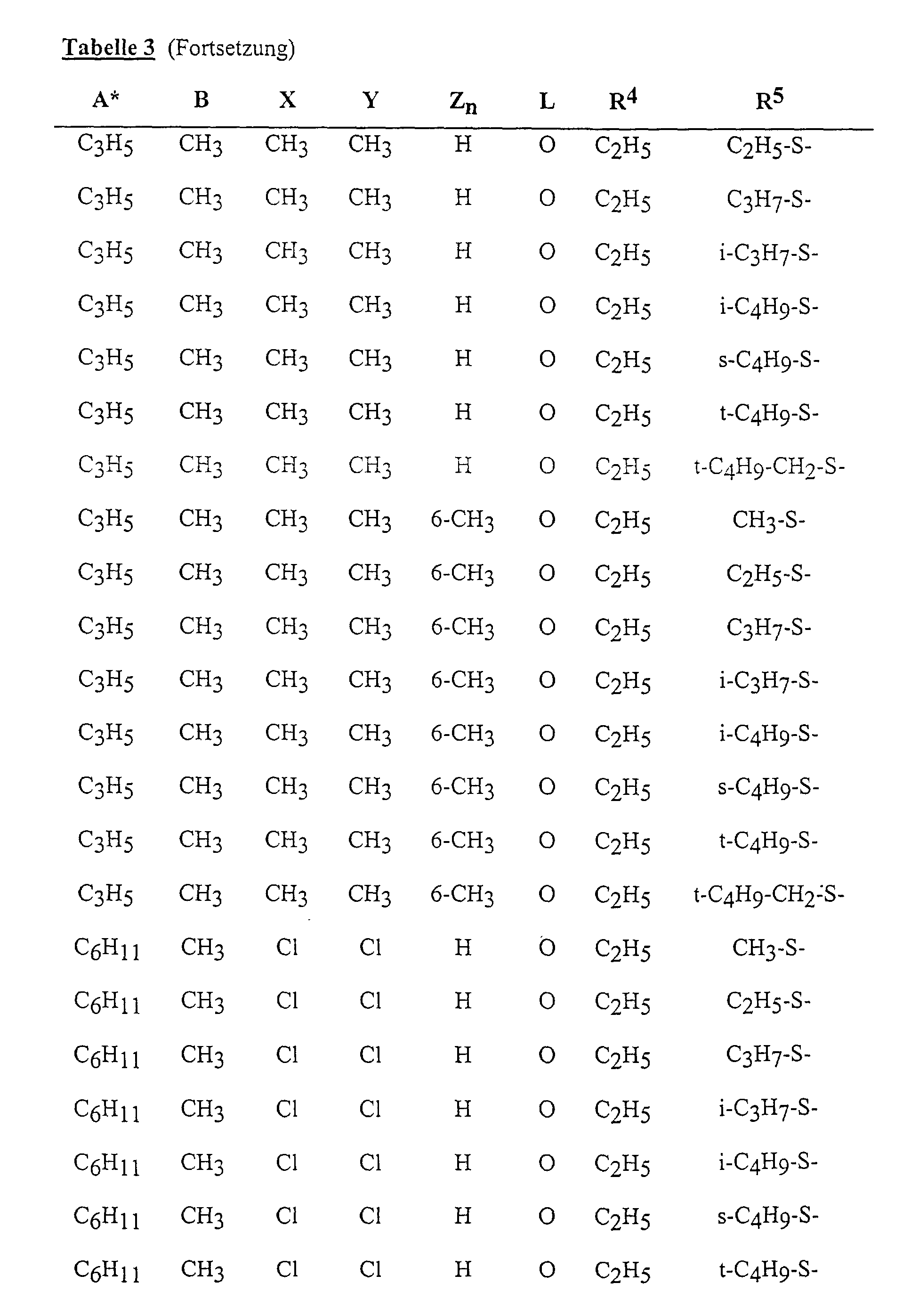

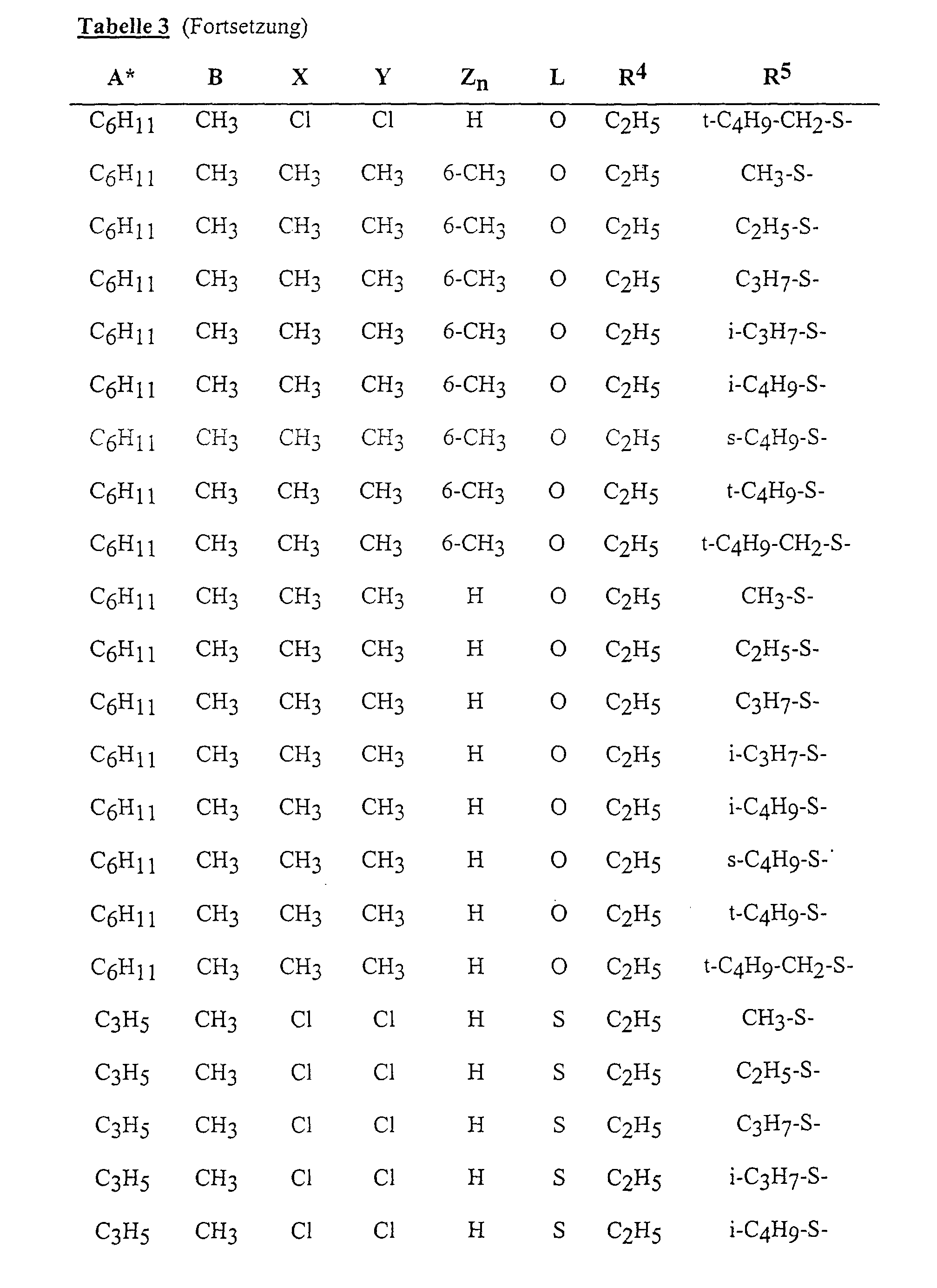

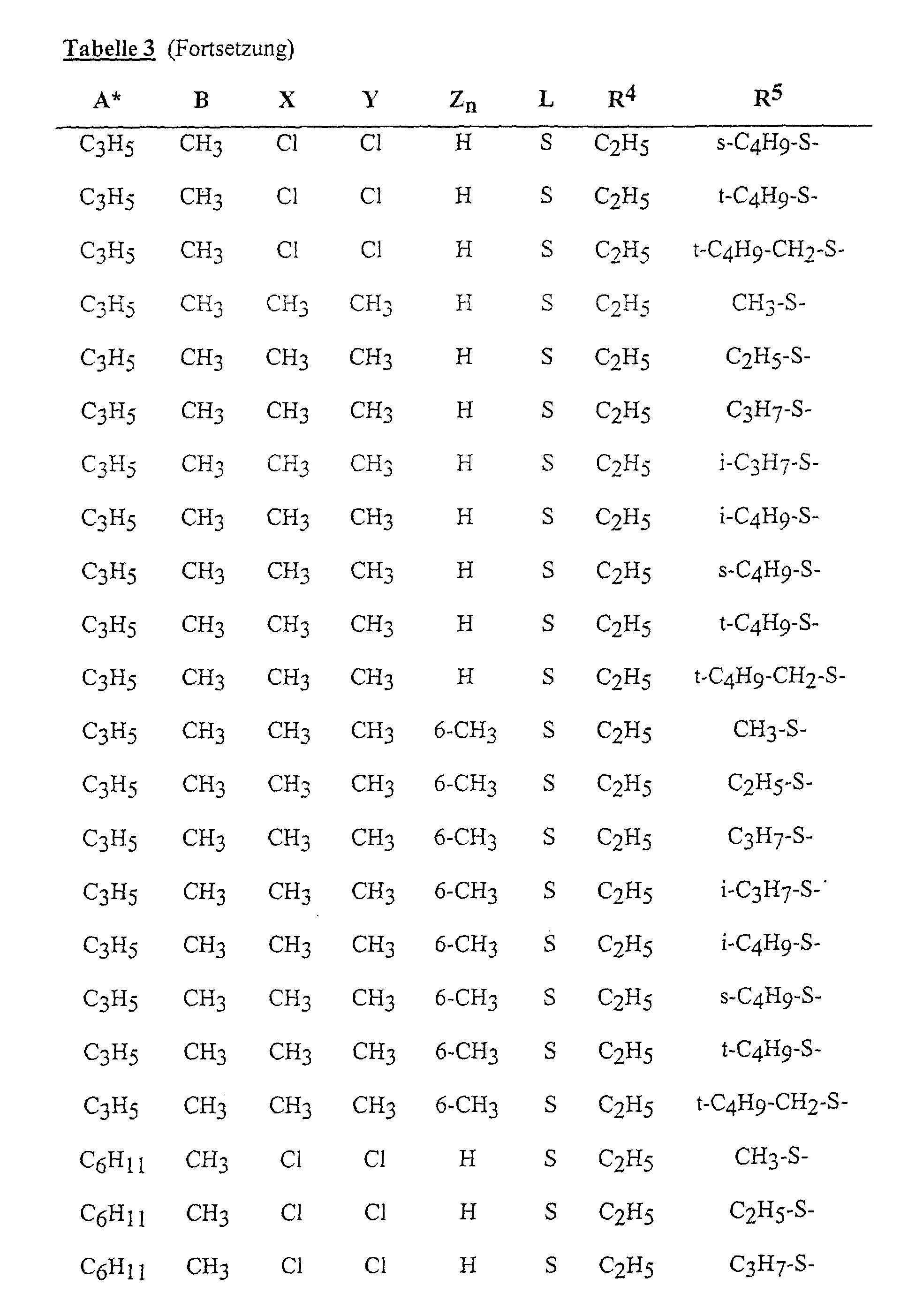

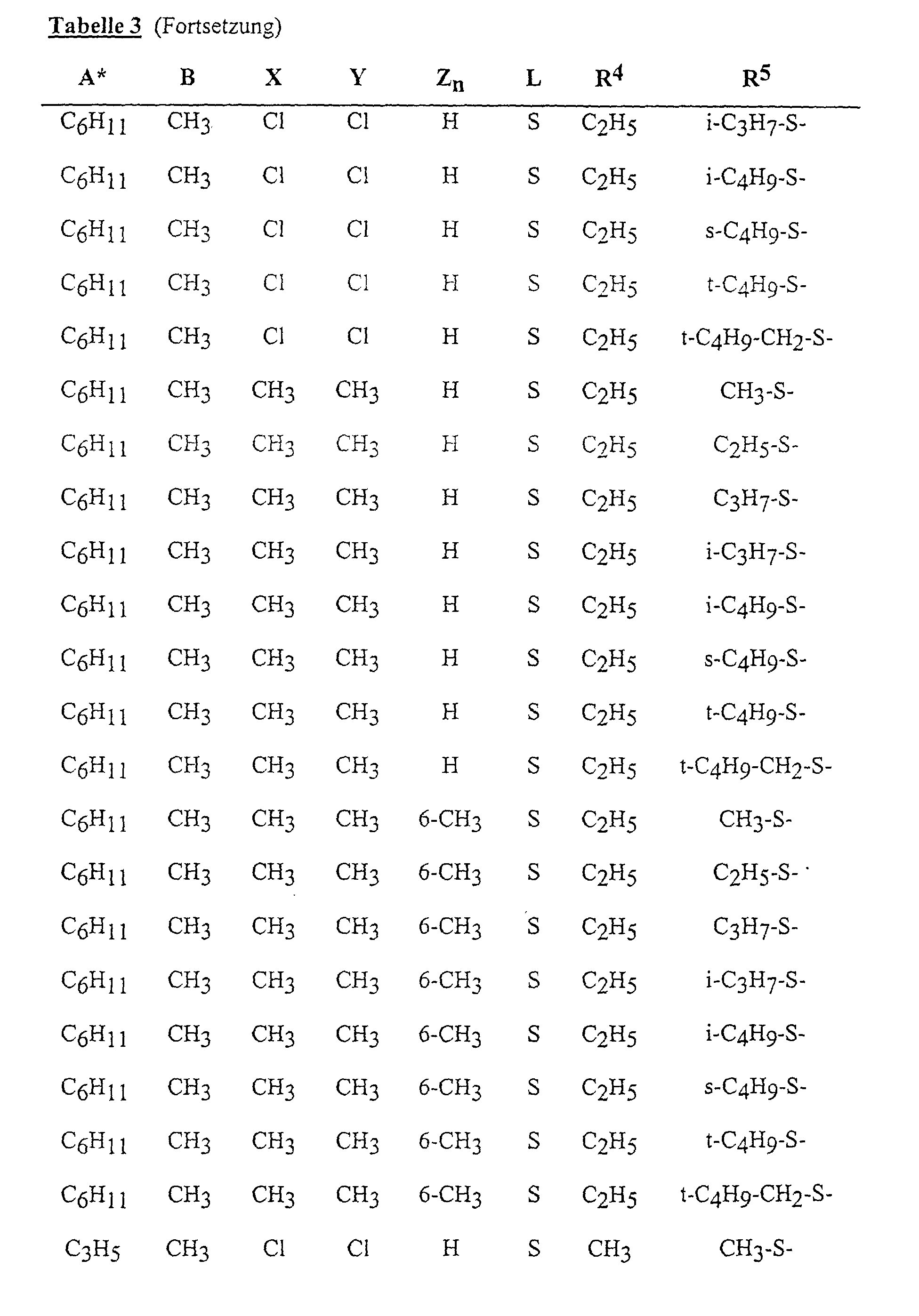

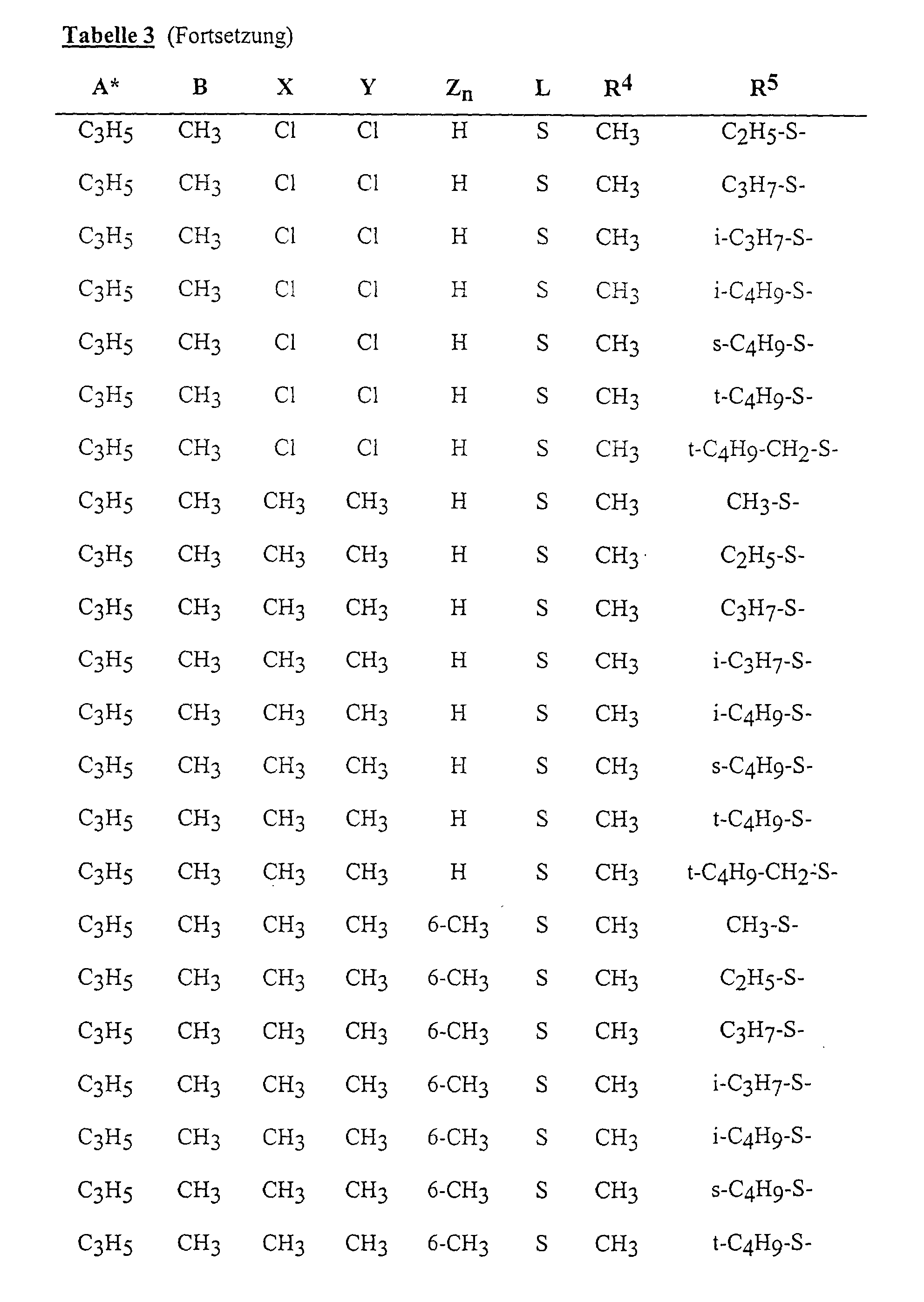

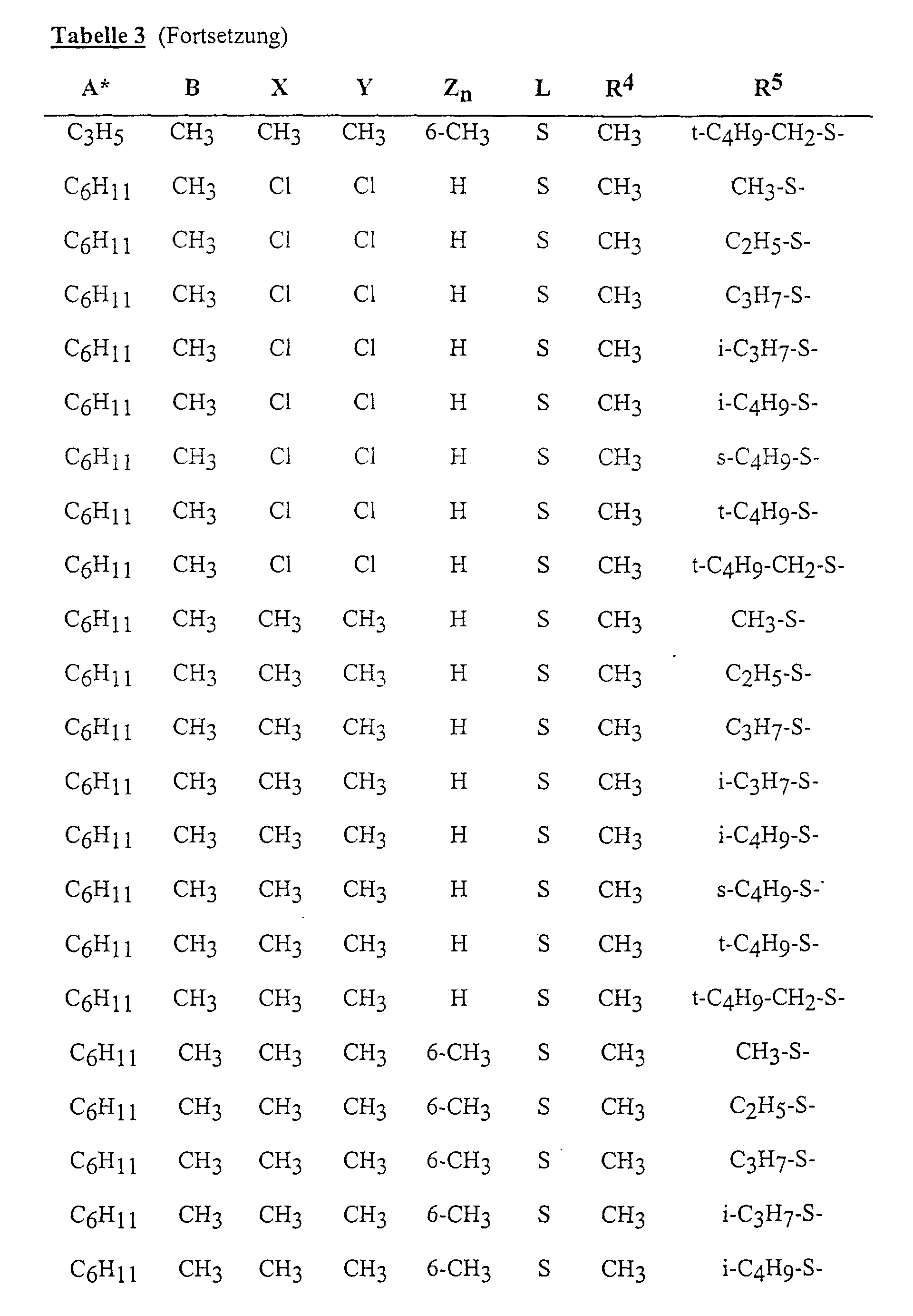

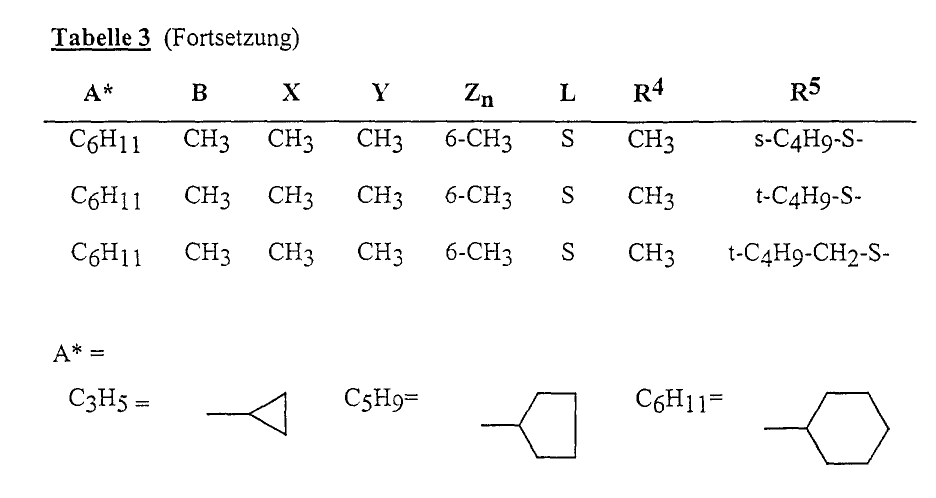

Im einzelnen seien außer den bei den Herstellungsbeispielen genannten Verbindungen die folgenden Verbindungen der Formel (Ig) genannt:

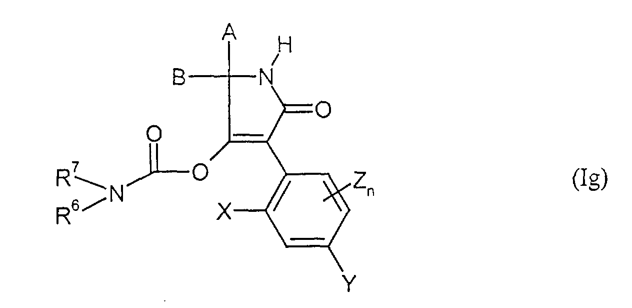

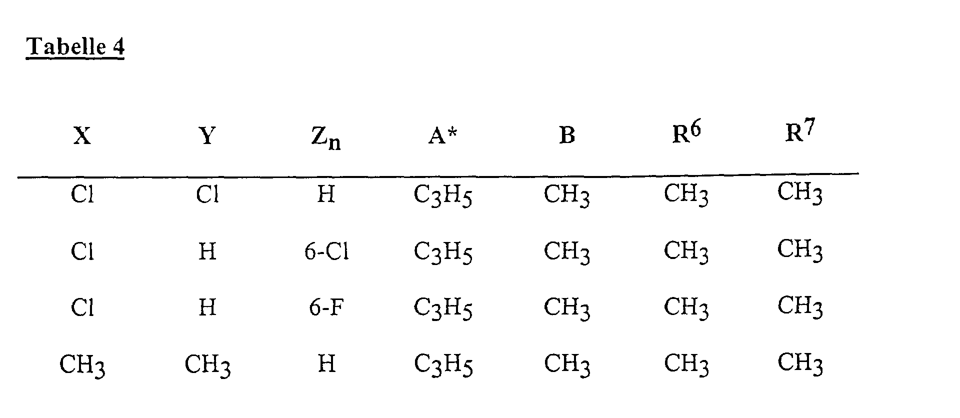

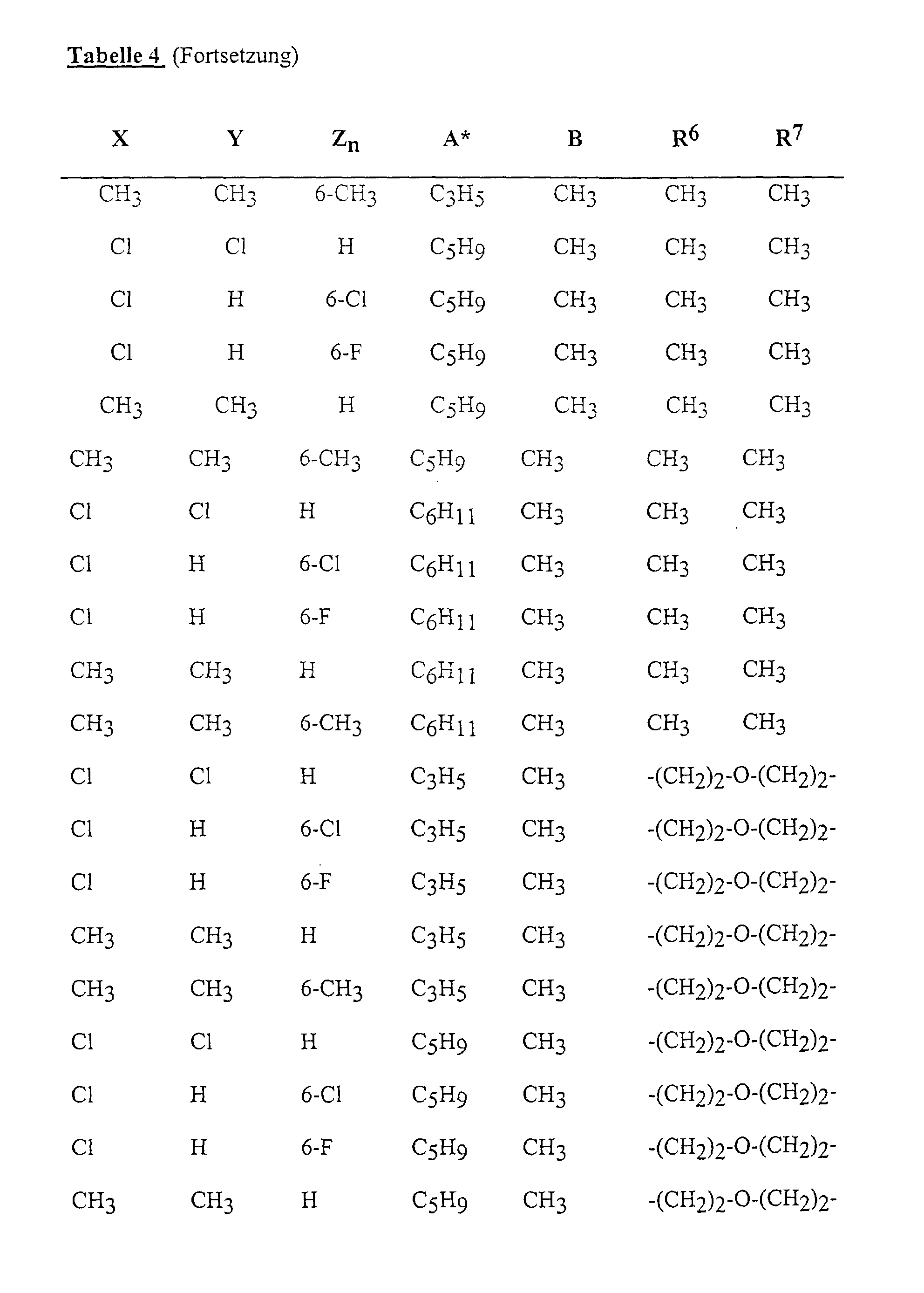

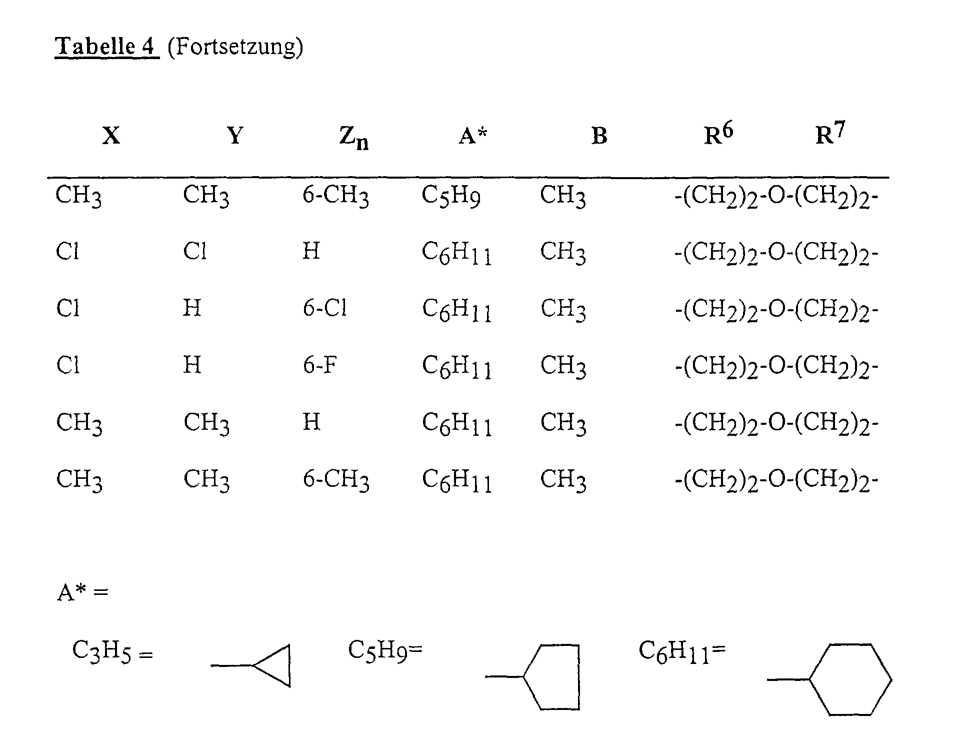

Verwendet man gemäß Verfahren (B) (Variante a) 3-(2,4,6-Trimethylphenyl)-5-cyclopropyl-5-methyl-pyrrolidin-2,4-dion und Pivaloylchlorid als Ausgangsstoffe, so kann der Verlauf des erfindungsgemäßen Verfahrens durch folgendes Reaktionsschema wiedergegeben werden:

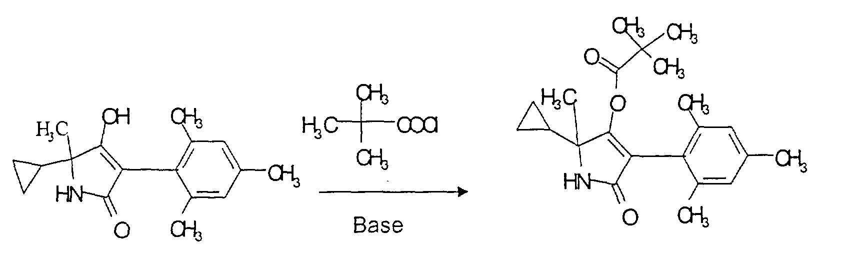

Verwendet man gemäß Verfahren B (Variante β) 3-(2,4,6-Trimethylphenyl)-5-cyclopentyl-5-ethyl-pyrrolidin-2,4-dion und Acetanhydrid als Ausgangsverbindungen, so kann der Verlauf des erfindungsgemäßen Verfahrens durch folgendes Reaktionsschema wiedergegeben werden:

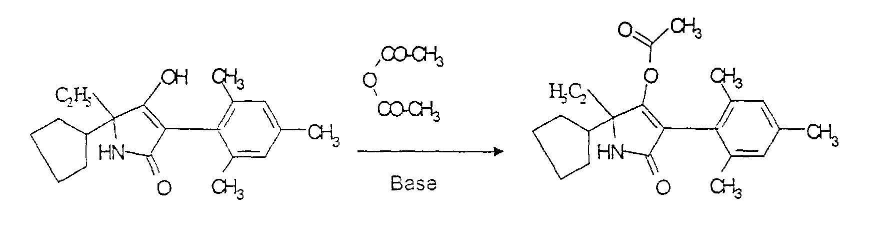

Verwendet man gemäß Verfahren (C) 3-(2,4,6-Trimethylphenyl)-5-[(4-methyl)-cyclohexyl]-5-methyl-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden.

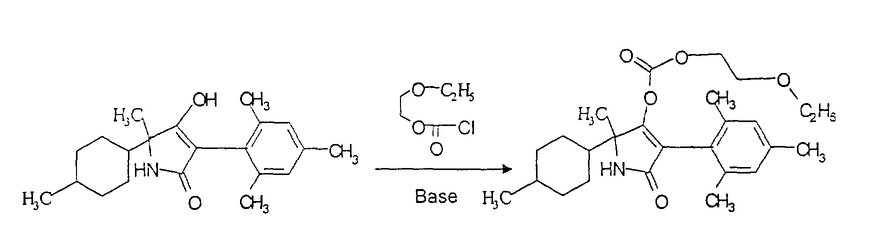

Verwendet man gemäß Verfahren (Dₐ) 3-(2,4,6-Trimethylphenyl)-5-cyclopentyl-5-methyl-pyrrolidin-2,4-dion und Chlormonothioameisensäuremethylester als Ausgangsprodukte, so kann der Reaktionsverlauf wie folgt wiedergegeben werden:

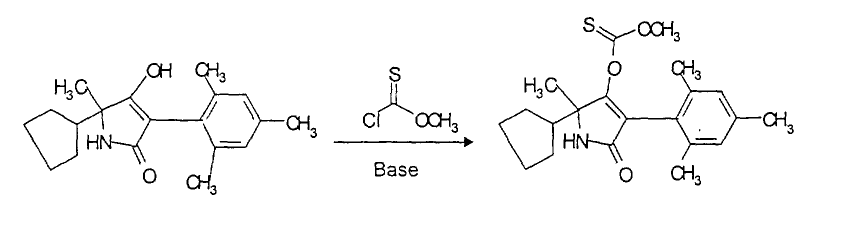

Verwendet man gemäß Verfahren (D_{β}) 3-(2,4,6-Trimethylphenyl)-5-[(3-methyl)-cyclohexyl]-5-ethyl-pyrrolidin-2,4-dion, Schwefelkohlenstoff und Methyliodid als Ausgangskomponenten, so kann der Reaktionsverlauf wie folgt wiedergegeben werden:

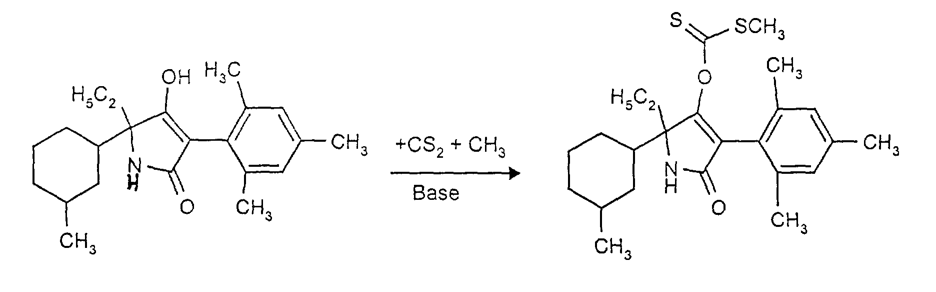

Verwendet man gemäß Verfahren (F) 3-(2,4-Dimethylphenyl)-5-cyclopropyl-5-methyl-pyrrolidin-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden:

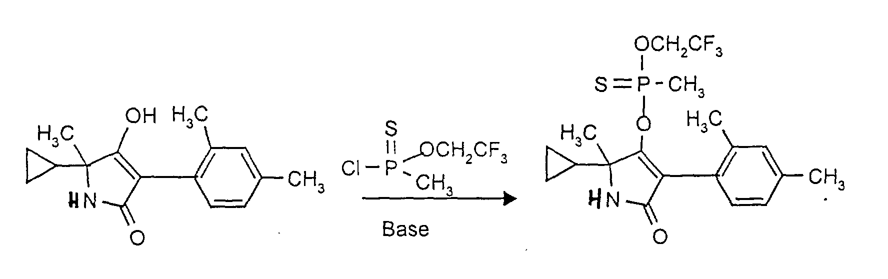

Verwendet man gemäß Verfahren (Hₐ) 3-(2,4,6-Trimethylphenyl)-5-[(4-methoxy)-cyclohexyl]-5-methyl-pyrrolidin-2,4-dion und Ethylisocyanat als Ausgangsprodukte, so kann der Reaktionsverlauf durch folgendes Schema wiedergegeben werden:

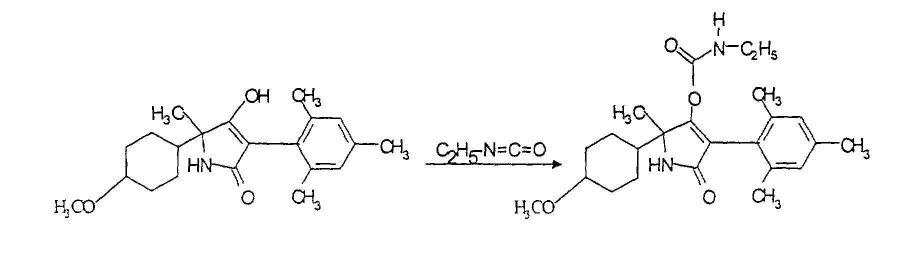

Verwendet man gemäß Verfahren (H_{b}) 3-(2,4,6-Trimethylphenyl)-5-cyclopentyl-5-methyl-pyrrolidin-2,4-dion und Dimethylcarbamidsäurechlorid als Ausgangsprodukte, so kann der Reaktionsverlauf durch folgendes Schema wiedergegeben werden:

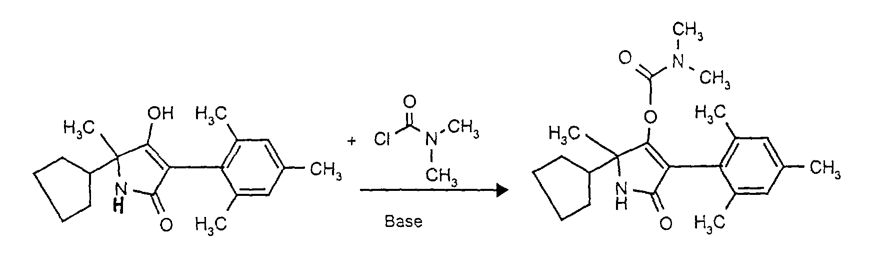

Die zur Durchführung der erfindungsgemäßen Verfahren (B), (C), (D), (F) und (H) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Alkylhalogenide der Formel (VII), Phosphorverbindungen der Formel (IX) und Isocyanate oder Carbamidsäurechlorid der Formel (XIII) sind allgemein bekannte Verbindungen der organischen bzw. anorganischen Chemie.

Das Verfahren (Ba) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Ba) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden,

Verwendet man die entsprechenden Carbonsäurehalogenide so kommen als Säurebindemittel bei der Umsetzung nach dem erfindungsgemäßen Verfahren (Ba) alle üblichen Säureakzeptoren in Betracht. Vorzugsweise verwendbar sind tertiäre Amine, wie Triethylamin, Pyridin, Diazabiyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können auch bei dem erfindungsgemäßen Verfahren (Ba) auch bei der Verwendung von Carbonsäurehalogeniden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Ba) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäureanhydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Die Reaktionstemperaturen können bei dem erfindungsgemäßen Verfahren (Bβ) auch bei der Verwendung von Carbonsäureanhydriden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Verwendet man die entsprechenden Chlorameisensäureester bzw. Chlorameisensäurethiolester so kommen als Säurebindemittel bei der Umsetzung nach dem erfindungsgemäßen Verfahren (C) alle üblichen Säureakzeptoren in Betracht.

Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) bei Verwendung der Chlorameisensäureester bzw. Chlorameisensäurethiolester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester bzw. Chlorameisensäurethiolester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren (D) setzt man pro Mol Ausgangsverbindung der Formel (Ia) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung Ia dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (D_{β}) setzt man pro Mol Ausgangsverbindung der Formel (Ia) die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus der Verbindung der Formel (Ia) durch Zusatz eines Deprotonierungsmittels (wie z.B. Kaliumtertiärbutylat oder Natriumhydrid) das entsprechende Salz herzustellen. Man setzt die Verbindung (Ia) solange mit Schwefelkohlenstoff um, bis die Bildung der Zwischenverbindung abgeschlossen ist, z.B. nach mehrstündigem Rühren bei Raumtemperatur.

Die weitere Umsetzung mit dem Alkylhalogenid der Formel (VII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Beim Herstellungsverfahren (F) setzt man zum Erhalt von Verbindungen der Struktur (Ie) auf 1 Mol der Verbindung (Ia), 1 bis 2, vorzugsweise 1 bis 1,3 Mol der Phosphorverbindung der Formel (IX) bei Temperaturen zwischen -40°C und 150°C, vorzugsweise zwischen -10 und 110°C um.

Als gegebenenfalls zugesetzte Verdünnungsmittel kommen aller inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Bei Herstellungsverfahren (Hₐ) setzt man pro Mol Ausgangsverbindung der Formel (Ia) ca. 1 Mol Isocyanat bzw. Isothiocyanat der Formel (XII) bei 0 bis 100°C, vorzugsweise bei 20 bis 50°C um.

Als gegebenenfalls zugesetzte Verdünnungsmittel kommen alle inerten organischen Lösungsmittel in frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (H_{b}) setzt man pro Mol Ausgangsverbindung der Formel (Ia) ca. 1 Mol Carbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Sulfone oder Sulfoxide.

Vorzusweise werden Dimethylsulfoxid, Tetrahydrofuran, Dimethylformamid oder Methylenchlorid eingesetzt

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werdern, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius,
Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium comi, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria. spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp. Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer. Beispielsweise zeigen sie eine hervorragende Wirksamkeit gegen Lucilia cuprina Larven.

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Milben, wie beispielsweise gegen die gemeine Spinnmilbe (Tetranychus urticae) einsetzen.

Die erfindungsgemäßen Wirkstoffe zeigen auch blattinsektizide Wirkung.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachauflaufverfahren. Sie können beispielsweise in Soja oder Zuckerrüben mit sehr gutem Erfolg zur Bekämpfung von Schadgräsem eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarb-stoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Weiterhin kommen 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); 2-[4-(2,4-Dichlorphenoxy)-phenoxy]-propionsäure, deren Methyl- oder deren Ethylester (DICLOFOPMETHYL); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-hamstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2- {[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIME-THALIN); o-(6-Chlor-3-phenylpyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure (THIAMETURON) in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

### Beispiel (Ib-1)

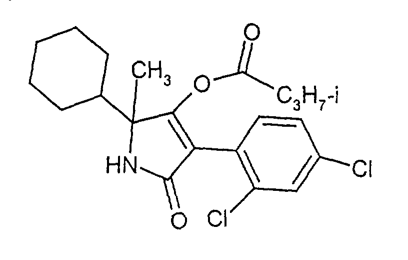

5,1 g (0,015 Mol) 3-(2,4-Dichlorphenyl)-5-cyclohexyl-5-methyl-pyrrolidin-2,4-dion-werden in 70 ml absolutem Dichlormethan suspendiert und mit 2,1 ml Triethylamin versetzt. Bei 0-10°C werden 1,58 ml Isobuttersäurechlorid in 5 ml absolutem Dichlormethan zugegeben. Das Ende der Reaktion wird dünnschichtchromatographisch ermittelt. Anschließend wird zweimal mit jeweils 100 ml 0,5 N Natronlauge gewaschen und die organische Phase über Magnesiumsulfat getrocknet. Der nach dem Abdampfen des Lösungsmittels erhaltene Rückstand wird aus Ether/n-Hexan 1:5 umkristallisiert.

Man erhält 4,4 g (72% der Theorie) 3-(2,4-Dichlorphenyl)-5-cyclohexyl-5-methyl-4-isobutyroxy-Δ3-pyrolin-2-on vom Schmelzpunkt Fp.: 138-140°C.

Analog zu Beispiel (Ib-1) und gemäß den allgemeinen Angaben zu den erfindungsgemäßen Verfahren werden die nachfolgend in Tabelle 9 aufgeführten Endprodukte der Formel (I-b) erhalten.

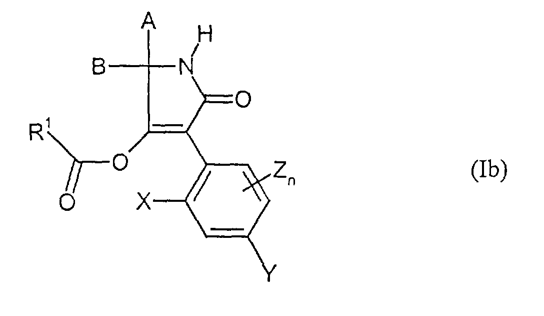

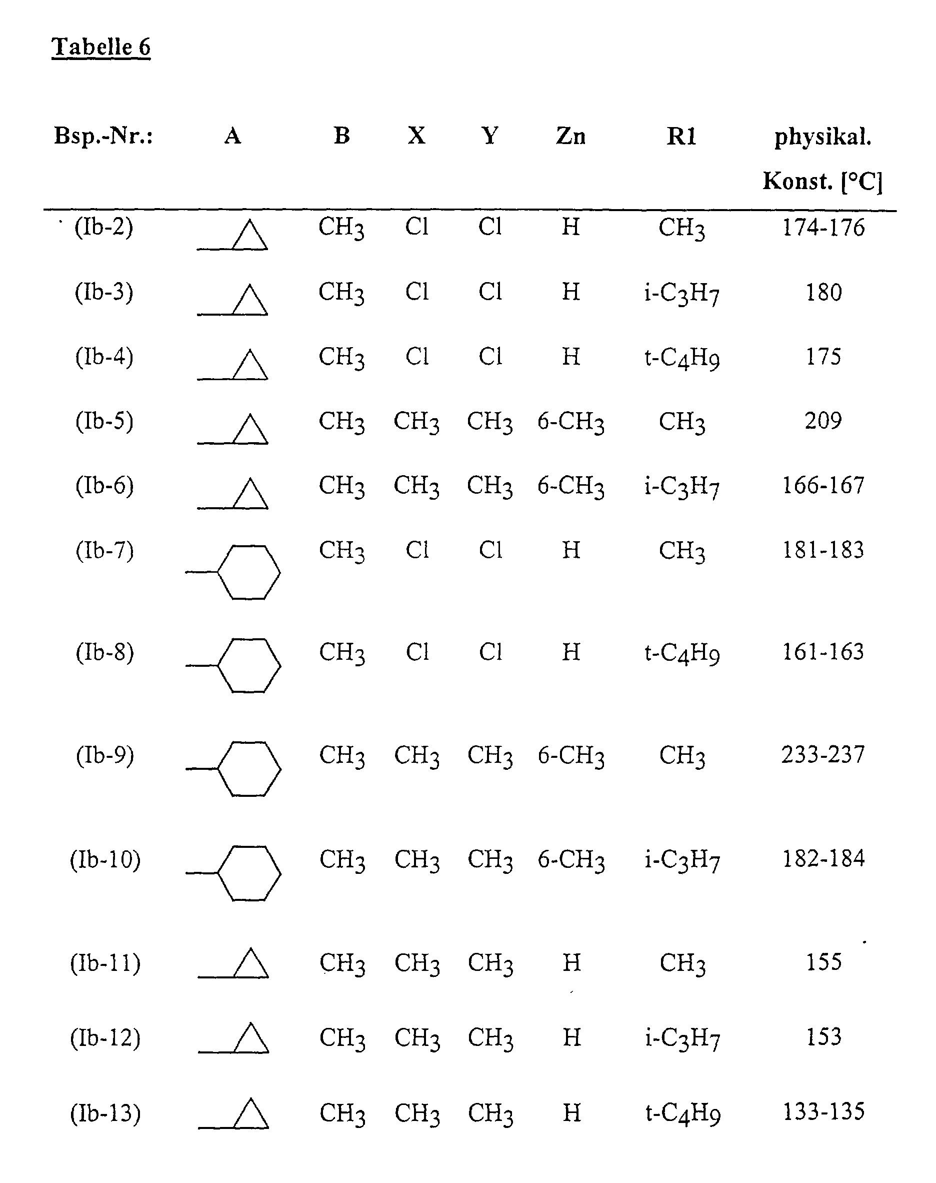

### Beispiel (Ic-1)

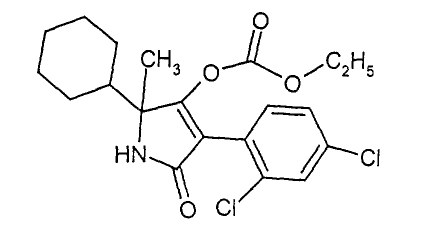

5,1 g (0,015 Mol) 3-(2,4-Dichlorphenyl)-5-cyclohexyl-5-methyl-pyrrolidin-2,4-dion werden in 70 ml absolutem Dichlormethan suspendiert und mit 2,1 ml Triethylamin versetzt. Bei 0-10°C werden 1,5 ml Chlorameisensäure-ethylester in 5 ml absolutem Dichlormethan zugegeben und der Ansatz bei Raumtemperatur weiter gerührt. Das Ende der Reaktion wird dünnschichtchromatographisch ermittelt. Anschließend wird zweimal mit jeweils 100 ml 0,5 N Natronlauge gewaschen und die organische Phase über Magnesiumsulfat getrocknet. Der nach dem Abdampfen des Lösungsmittels erhaltene Rückstand wird aus Ether/n-Hexan (1:5) umkristallisiert
Man erhät 4,6 g (74 % der Theorie) Kohlensäure-O-ethylester-O-[3-(2,4-Dichlorphenyl)-5-cyclohexyl-5-methyl-Δ3-pyrrolidin-4-yl-2-on] vom Schmelzpunkt Fp.: 175-176°C.

Analog zu Beispiel (Ic-1) und gemäß den allgemeinen Angaben zu den erfindungsgemäßen Verfahren werden die nachfolgend in Tabelle 10 aufgeführten Endprodukte der Formel (Ic) erhalten

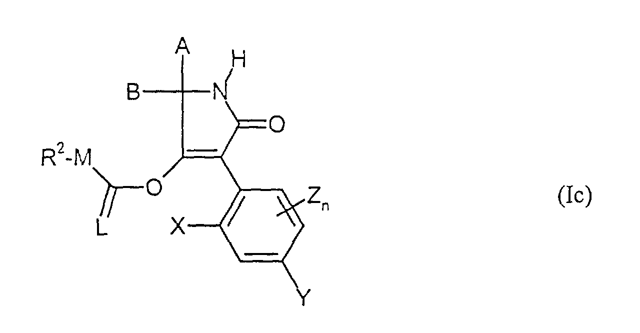

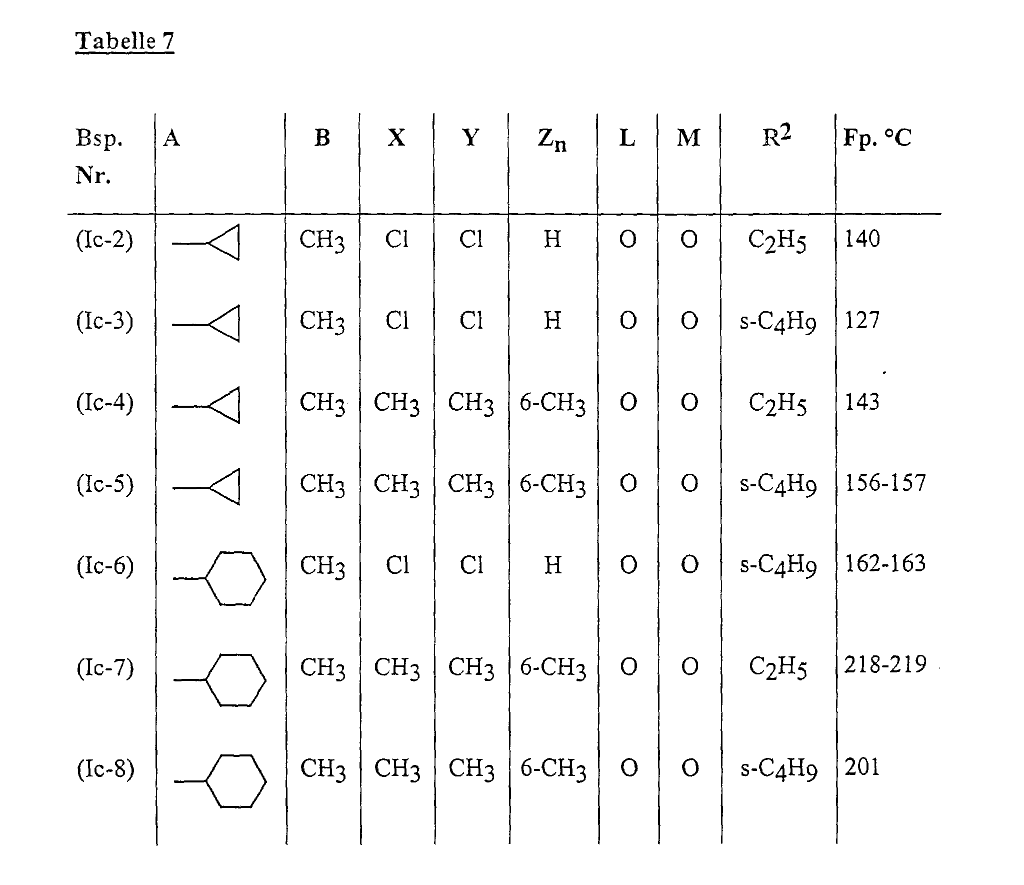

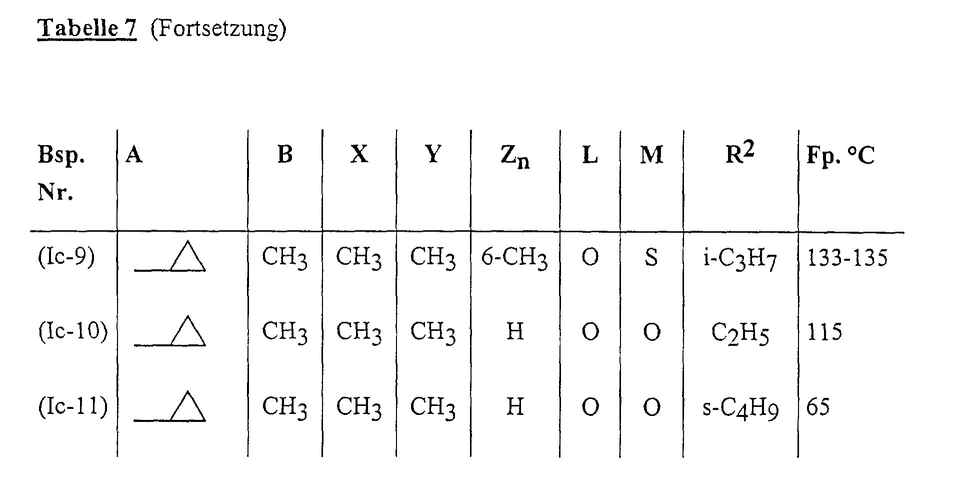

### Beispiel (Ie-1)

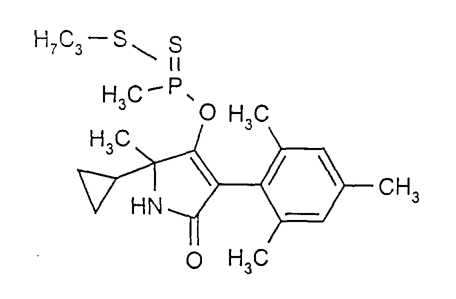

3 g (11 mmol) 3-(2.4.6-Trimethylphenyl)-5-cyclopropyl-5-methyl-pyrrolidin-2,4-dion werden in 20 ml absolutem Tetrahydrofuran suspendiert und mit 1,7 ml Triethylamin versetzt. Nach Zugabe von 2,2 g Methyl-propylmercapto-thiophosphonsäurechlorid erwärmt man 24 h auf 50°C. Das Lösungsmittel wird abgedampft und der Rückstand an Kieselgel mit n-Hexan/Aceton 9:1 als Fließmittel chromatographiert. Man erhält 1,7 g (3,7 % der Theorie) der oben gezeigten Verbindung vom Schmp. 143°C.

### Beispiel (Ie-2)

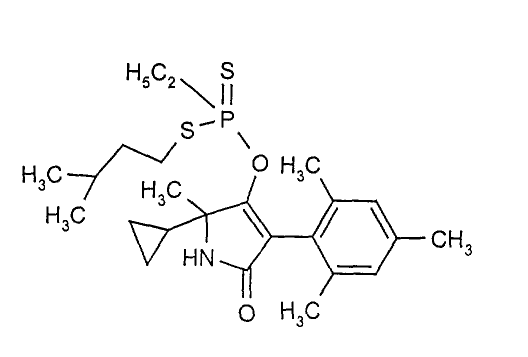

Analog zu Beispiel (Ie-1) erhält man die oben abgebildete Verbindung vom Schmp. 100°C.

### Beispiel (Ig-1)

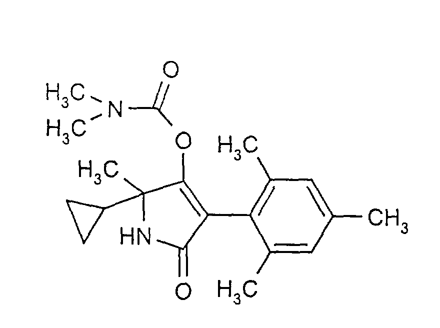

4,86 g (0,02 Mol) 3-(2,4,6-Trimethylphenyl)-5-cyclopropyl-5-methyl-pyrrolidin-2,4-dion werden in 70 ml absolutem Tetrahydrofuran suspendiert und mit 2,8 ml Triethylamin versetzt. Bei 0 bis 10°C werden 1,84 ml Dimethylcarbamidsäurechlorid in 5 ml absolutem Tetrahydrofuran zugetropft. Nach Zugabe von 20 mg 4-N,N-Dimethylaminopyridin wird unter dünnschichtchromatographischer Kontrolle unter Rückfluß gekocht. Das Lösungsmittel wird im Vakuum abgedampft, der Rückstand in CH₂CH₂ aufgenommen, 2 mal mit 0,5 N Natronlauge gewaschen, die organische Phase mit Magnesiumsulfat getrocknet und das Lösungsmittel abgedampft. Der Rückstand wird aus Methyl-tert.-butylether/n-Hexan umkristallisiert. Dabei erhält man 3,1 g (45 % der Theorie) 4-Dimethylcarbamoyloxy-5-cyclopropyl-5-methyl-3-(2,4,6-trimethylphenyl)-Δ3-pyrrolin-2-on vom Schmp. 201-205°C.

### Beispiel (Ig-2)

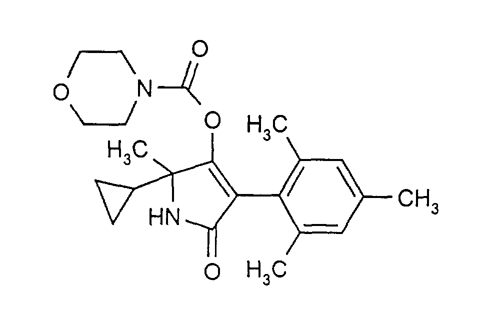

Analog zu Beispiel (Ig-1) erhält man 4-Morpholincarbamoyloxy-5-cyclopropyl-5-methyl-3 -(2,4,6-trimethylphenyl)-A3-pyrrolin-2-on vom Schmp. 137-141°C.

### Herstellung der Ausgangsverbindungen:

### Beispiel (II-1)

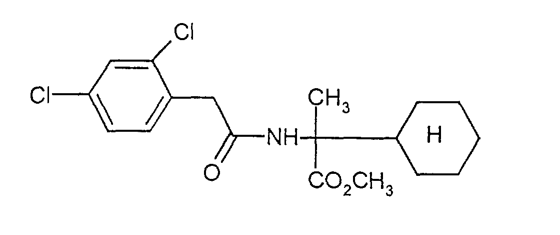

Zu 128 g (1,31 Mol) konzentrierter Schwefelsäure gibt man unter Rühren und Eiskühlung tropfenweise 89,2 g (0,263 Mol) N-(1-cyano-1-methyl-cyclohexylmethyl)-2-(2,4-dichlorphenyl)-acetamid, in 270 ml Dichlormethan gelöst, wobei sich die Temperatur der Reaktionsmischung auf 40°C erwärmt und rührt nach beendeter Zugabe weitere 2 Stunden bei 40°C, bis die Dichlormethanphase der Reaktionsmischung farblos geworden ist. Anschließend gibt man tropfenweise unter Eiskühlung 184 ml absolutes Methanol zu, wobei sich die Reaktionsmischung bis 40°C erwärmt und rührt weitere 6 Stunden bei 40-50°C. Zur Aufarbeitung gibt man die Reaktionsmischung unter Rühren in 1500 g Eis, extrahiert mit Dichlormethan, wäscht die vereinigten organischen Phasen mit wäßriger Natriumhydrogencarbonatlösung säurefrei, trocknet über Magnesiumsulfat und entfernt das Lösungsmittel im Vakuum.

Man erhält 83,6 g (85 % der Theorie) N-(2,4-Dichlorphenyl-acetyl)-2-cyclohexylalanin-methylester vom Schmelzpunkt Fp.: 107-108°C.

### Beispiel (II-2)

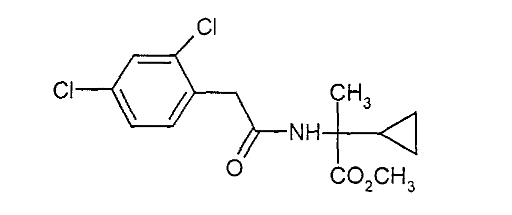

Analog zu Beispiel (II-1) erhält man N-(2,4-Dichlorphenyl-acetyl)-2-cyclopropylalaninmethylester vom Schmelzpunkt Fp.: 81°C.

### Anwendungsbeispiele:

In den folgenden Anwendungsbeispielen wurde die nachfolgend aufgeführten Verbindungen als Vergleichssubstanzen eingesetzt:

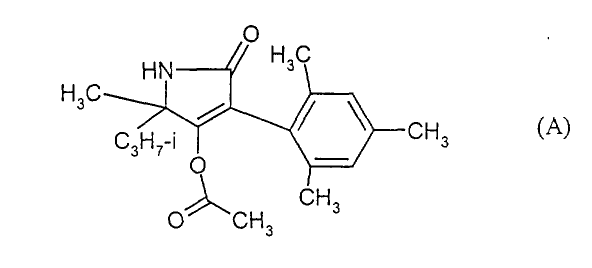

3-(2,4,6-Trimethylphenyl)-5-methyl-5-isopropyl-4-acetoxy-A3-pyrrolin-2-on bekannt aus EP 456 063.

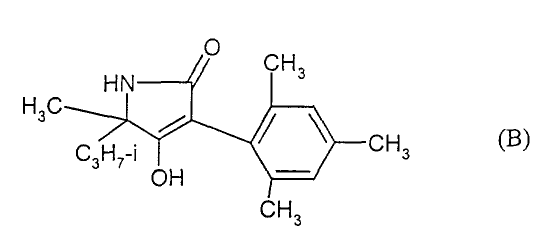

3-(2,4,6-Trimethylphenyl)-5-methyl-5-isopropyl-pyrrolidin-2,4-dion bekannt aus EP 456 063.

### Beispiel A

### Pre-emergence Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei erhält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- 0% =: keine Wirkung (wie unbehandelte Kontrolle)
- 100%=: totale Vernichtung

Bei diesem Test wurden mit einer beispielhaften Aufwandmenge von 125 g/ha bei einer sehr guten Verträglichkeit durch Zuckerrüben die folgenden Ergebnisse erhalten:

| Pflanze | % Wirkung | Verbindung des Herstellungsbeispieles Nr. |
|---|---|---|
| Digitaria | ≥80 | Ia-3, Ib-5, Ib-6, Ic-4 |
| Alopecurus | ≥80 | Ia-3, Ib-5, Ib-6, Ic-4 |
| Lolium | ≥90 | Ia-3, Ib-5, Ib-6, Ic-4 |

### Beispiel B

### Post-emergence Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben, so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle. Es bedeuten:
- 0%=: keine Wirkung (wie unbehandelte Kontrolle)
- 100%=: totale Vernichtung

Bei diesem Test wurden mit einer beispielhaften Aufwandmenge von 125 g/ha bei einer sehr guten Verträglichkeit durch Soja die folgenden Ergebnisse erhalten:

| Pflanze | % Wirkung | Verbindung des Herstellungsbeispieles Nr. |
|---|---|---|
| Cynodon | ≥30 | Ia-2, Ia-3, Ib-2, Ib-3, Ib-5, Ib-6, Ic-3, Ic-4 |
| Echinochloa | ≥80 | Ia-2, Ia-3, Ib-2, Ib-3, Ib-5, Ib-6, Ic-3, Ic-4 |
| Setaria | ≥80 | Ia-2, Ia-3, Ib-2, Ib-3, Ib-5, Ib-6, Ic-3, Ic-4 |

### Beispiel C

### Tetranychus-Test (OP-resistent)

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test bewirkte z.B. die Verbindung gemäß Herstellungsbeispiel (Ic-3) bei einer beispielhaften Wirkstoffkonzentration von 0,02 % eine Abtötung von 100 % nach 7 Tagen.

## Patentansprüche

1. 1-H-3-Phenyl-5-cycloalkylpyrrolidon-2,4-dione der Formel (I) 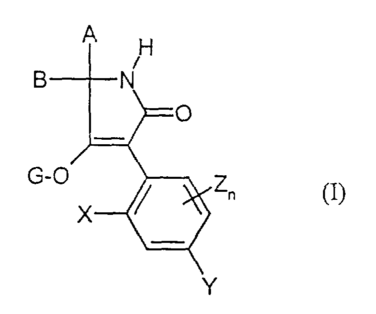 in welcher
A für C₃-C₆-Cycloalkyl steht,
B für Methyl, Ethyl, Propyl, i-Propyl, Butyl, i-Butyl, s-Butyl oder t-Butyl steht,
X für Methyl, Ethyl, Propyl, i-Propyl, Fluor, Chlor oder Brom, steht,
Y für Methyl, Ethyl, Propyl, i-Propyl, Butyl, i-Butyl, tert.-Butyl, Fluor, Chlor oder Brom steht,
Z für Methyl, Ethyl, Propyl, i-Propyl, Butyl, i-Butyl oder tert.-Butyl, steht,
n für 0 oder 1 steht,
G für die Gruppen 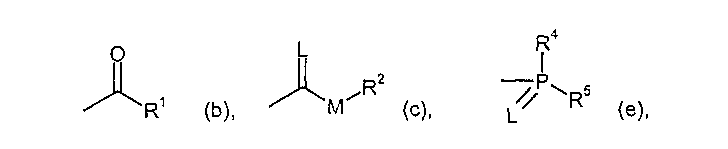 oder 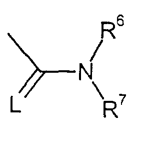 (g), steht,
in welchen
L und M für Sauerstoff und/oder Schwefel stehen,
R¹ für C₁-C₁₄-Alkyl steht,
R² für C₁-C₁₄-Alkyl steht,
R⁴ und R⁵ unabhängig voneinander für C₁-C₄-Alkyl oder C₁-C₄-Alkylthio stehen,
R⁶ und R⁷ unabhängig voneinander für C₁-C₄-Alkyl oder zusammen mit dem N-Atom, an das sie gebunden sind, für einen gegebenenfalls durch Sauerstoff oder Schwefel unterbrochenen C₄-C₆-Alkylenring stehen.

2. 1-H-3-Phenyl-5-cycloalkylpyrrolidin-2,4-dione gemäß Anspruch 1, mit folgenden Formeln 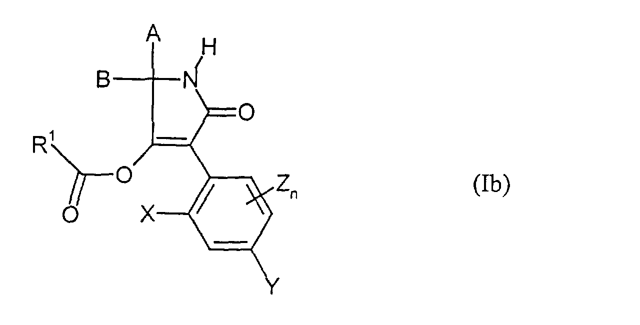 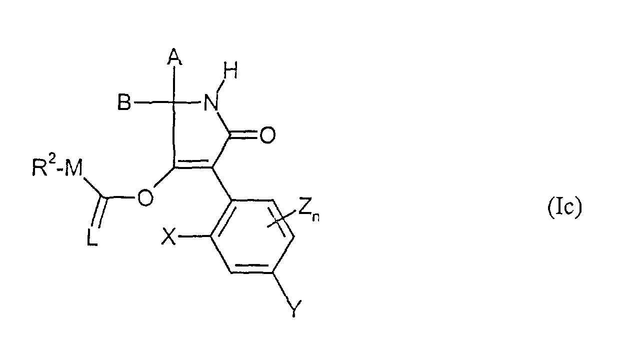 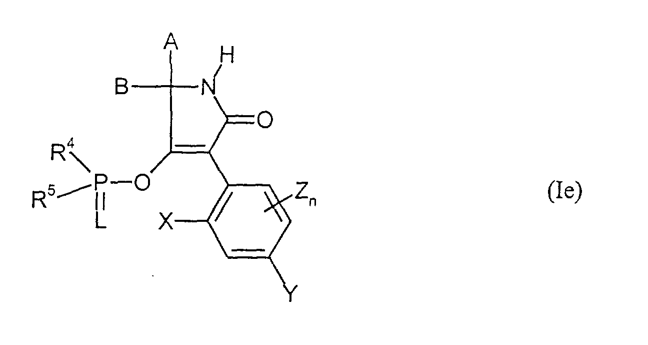 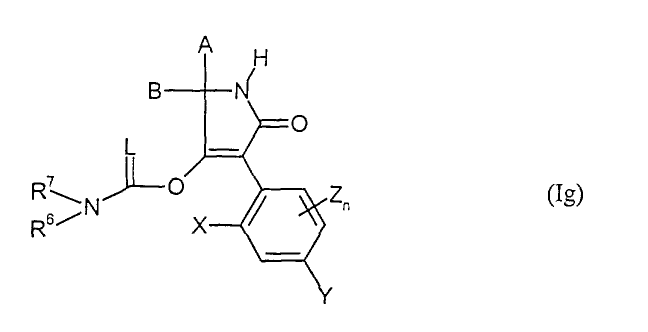 worin
A, B, L, M, X, Y, Zₙ, R¹, R², R⁴, R⁵, R⁶ und R⁷
die in Anspruch 1 angegebenen Bedeutungen besitzen.

3. Verfahren zur Herstellung der 1-H-3-Phenyl-5-cycloalkylpyrrolidin-2,4-dione gemäß Anspruch 1, **dadurch gekennzeichnet, dass** man
(B) für den Fall von Verbindungen der Formel (Ib) 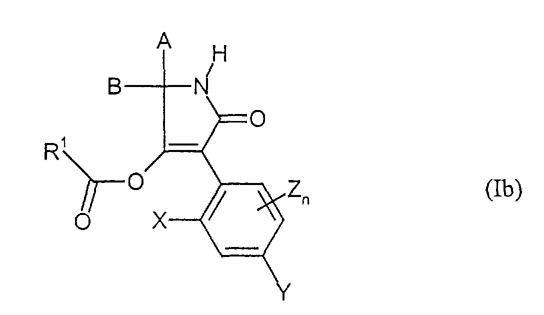 in welcher
A, B, X, Y, Z, R¹ und n die in Anspruch 1 angegebene Bedeutung haben,
Verbindungen der Formel (Ia), 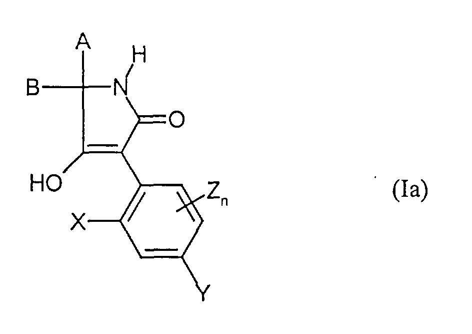 in welcher
A, B, X, Y, Z und n die oben angegebene Bedeutung haben,
a) mit Säurehalogeniden der allgemeinen Formel (III) 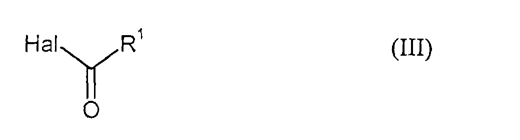 in welcher
R¹ die in Anspruch 1 angegebene Bedeutung hat und
Hal für Halogen, insbesondere Chlor und Brom steht,
gegebenenfalls in Gegenwart eines Verdünnungsmittels und gegebenenfalls in Gegenwart eines Säurebindemittels umsetzt
oder
β) mit Carbonsäureanhydriden der allgemeinen Formel (IV)
R¹-CO-O-CO-R¹ (IV)
in welcher
R¹ die in Anspruch angegebene Bedeutung hat,
gegebenenfalls in Gegenwart eines Verdünnungsmittels und gegebenenfalls in Gegenwart eines Säurebindemittels,
umsetzt;
oder
(C) für den Fall von Verbindungen der Formel (Ic-1) 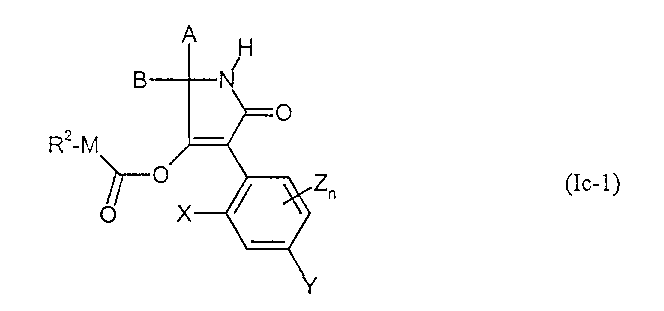 in welcher
A, B, X, Y, Z, R² und n die in Anspruch 1 angegebene Bedeutung haben,
und
M für Sauerstoff oder Schwefel steht,
Verbindungen der Formel (Ia) 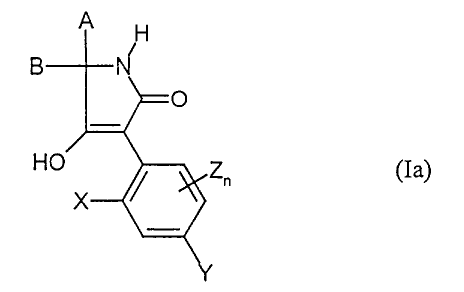 in welcher
A, B, X, Y, Z und n die in Anspruch 1 angegebene Bedeutung haben,
mit Chlorameisensäureester oder Chlorameisensäurethiolester der allgemeinen Formel (V)
R²-M-CO-Cl (V)
in welcher
R² und M die in Anspruch 1 angegebene Bedeutung haben,
gegebenenfalls in Gegenwart eines Verdünnungsmittels und gegebenenfalls in Gegenwart eines Säurebindemittels umsetzt;
oder
(D) für den Fall von Verbindungen der Formel (Ic-2) 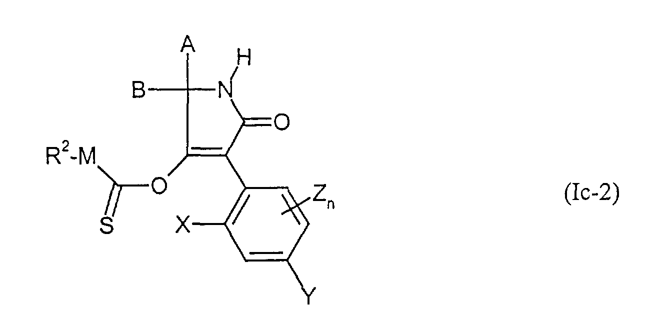 in welcher
A, B, R², X, Y, Z und n die in Anspruch 1 angegebene Bedeutung haben
und
M für Sauerstoff oder Schwefel steht,
Verbindungen der Formel (Ia) 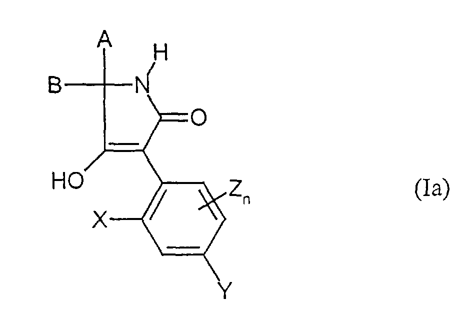 in welcher
A, B, X, Y, Z und n die in Anspruch 1 angegebene Bedeutung haben,
(a) mit Chlormonothioameisensäurestern oder Chlordithioameisensäureesteren der allgemeinen Formel (VI) 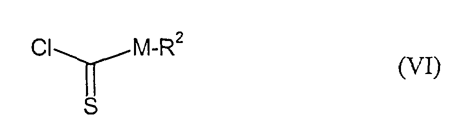 in welcher
M und R² die in Anspruch 1 angegebene Bedeutung haben,
gegebenenfalls in Gegenwart eines Verdünnungsmittels und
gegebenenfalls in Gegenwart eines Säurebindemittels umsetzt,
oder
β) mit Schwefelkohlenstoff und anschließend mit Alkylhalogeniden der allgemeinen Formel (VII)
R²-Hal (VII)
in welcher
R² die in Anspruch 1 angegebene Bedeutung hat
und
Hal für Chlor, Brom, Iod steht,
gegebenenfalls in Gegenwart eines Verdünnungsmittels umsetzt;
oder
(F) für den Fall von Verbindungen der Formel (Ie) 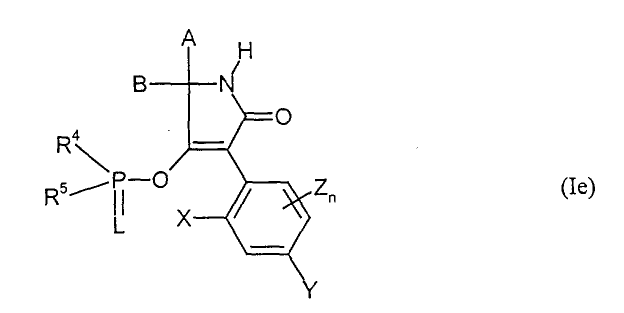 in welcher
A, B, L, X, Y, Z, R⁴, R⁵ und n die in Anspruch 1 angegebene Bedeutung haben,
1-H-3-Phenyl-5-cycloalkylpyrrolidin-2,4-dione der Formel (Ia) bzw. deren Enole 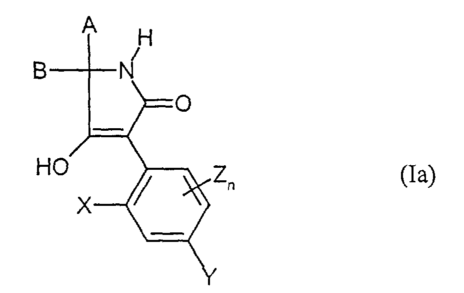 in welcher
A, B, X, Y, Z und n die in Anspruch 1 angegebene Bedeutung haben,
mit Phosphorverbindungen der allgemeinen Formel (IX) 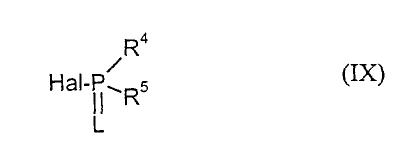 in welcher
L, R⁴ und R⁵ die in Anspruch 1 angegebene Bedeutung haben
und
Hal für Halogen, insbesondere Chlor oder Brom steht,
gegebenenfalls in Gegenwart eines Verdünnungsmittels und gegebenenfalls in Gegenwart eines Säurebindemittels umsetzt;
oder
(H) für den Fall von Verbindungen der Formel (Ig) 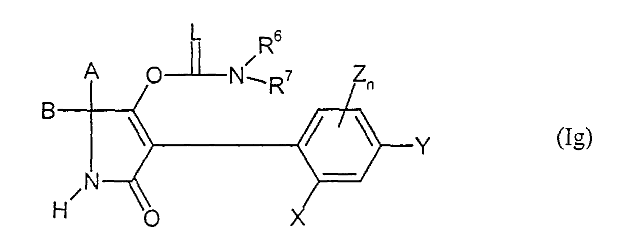 in welcher
A, B, L, X, Y, Z, R⁶, R⁷ und n die in Anspruch 1 angegebene Bedeutung haben,
Verbindungen der Formel (Ia) 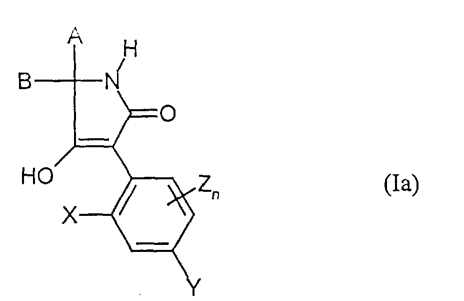 in welcher
A, B, X, Y, Z und n die in Anspruch 1 angegebene Bedeutung haben
a) mit Verbindungen der allgemeinen Formel (XII)
R⁶-N=C=L (XII)
in welcher
L und R⁶ die in Anspruch 1 angegebene Bedeutung haben
gegebenenfalls in Gegenwart eines Verdünnungsmittels und
gegebenenfalls in Gegenwart eines Katalysators
oder
b) mit Carbamidsäurechloriden oder Thiocarbamidsäurechloriden der allgemeinen Formel (XIII) 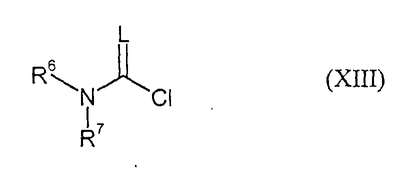 in welcher
L, R⁶ und R⁷ die in Anspruch 1 angegebene Bedeutung haben
gegebenenfalls in Gegenwart eines Verdünnungsmittels und
gegebenenfalls in Gegenwart eines Säurebindemittels,
umsetzt.

4. Schädlingsbekämpfungsmittel und Herbizide, **gekennzeichnet durch** einen Gehalt an mindestens einem 1-H-3-Phenyl-5-cycloalkylpyrrolidin-2,4-dione der Formel (I), gemäß Anspruch 1.

5. Verwendung von 1-H-3-Phenyl-5-cycloalkylpyrrolidin-2,4-dione der Formel (I) gemäß Anspruch 1, zur Bekämpfung von Schädlingen und unerwünschtem Pflanzenwuchs.

6. Verfahren zur Bekämpfung von Schädlingen, **dadurch gekennzeichnet, dass** man 1-H-3-Phenyl-5-cycloalkylpyrrolidin-2,4-dione der Formel (I) gemäß Anspruch 1 auf Schädlinge, unerwünschte Pflanzen und/oder ihren Lebensraum einwirken lässt.

7. Verfahren zur Herstellung von Schädlingsbekämpfungsmitteln, **dadurch gekennzeichnet, dass** man 1-H-3-Phenyl-5-cycloalkylpyrrolidin-2,4-dione der Formel (I) gemäß Anspruch 1 mit Streckmitteln und/oder oberflächenaktiven Mitteln vermischt.

## Claims

1. 1-H-3-Phenyl-5-cycloalkylpyrrolidine-2,4-diones of the formula (I) 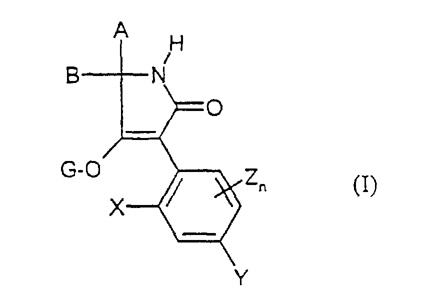 in which
A represents C₃-C₆-cycloalkyl,
B represents methyl, ethyl, propyl, i-propyl, butyl, i-butyl, s-butyl or t-butyl,
X represents methyl, ethyl, propyl, i-propyl, fluorine, chlorine or bromine,
Y represents methyl, ethyl, propyl, i-propyl, butyl, i-butyl, tert-butyl, fluorine, chlorine or bromine,
Z represents methyl, ethyl, propyl, i-propyl, butyl, i-butyl or tert-butyl,
n represents 0 or 1,
G represents the groups 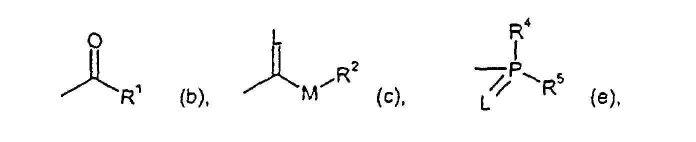 or 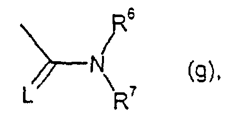 in which
L and M represent oxygen and/or sulphur,
R¹ represents C₁-C₁₄-alkyl,
R² represents C₁-C₁₄-alkyl,
R⁴ and R⁵ independently of one another represent C₁-C₄-alkyl or C₁-C₄-alkylthio,
R⁶ and R⁷ independently of one another represent C₁-C₄-alkyl or together with the N atom to which they are attached represent a C₄-C₆-alkylene ring which is optionally interrupted by oxygen or sulphur.

2. 1-H-3-Phenyl-5-cycloalkylpyrrolidine-2,4-diones according to Claim 1, of the following formulae 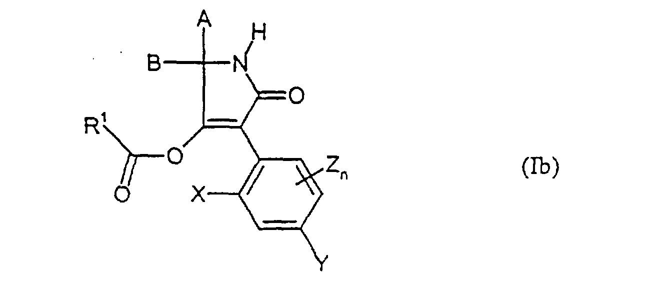 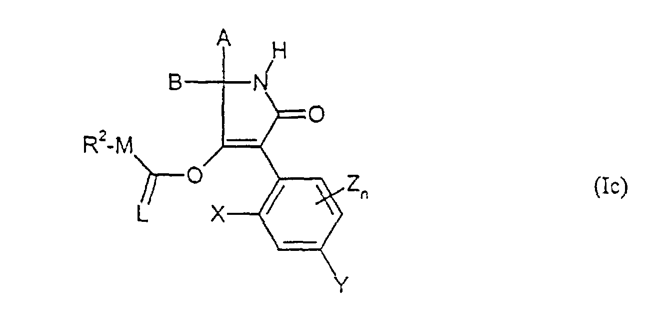 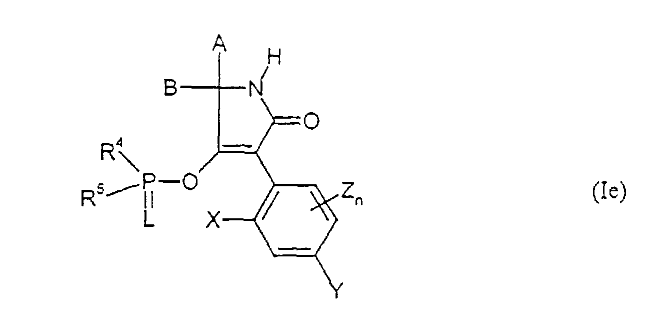 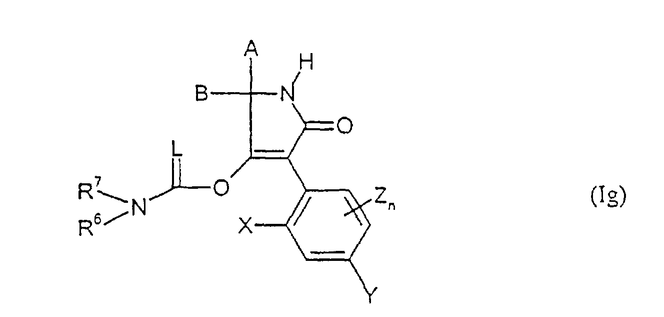 in which
A, B, L, M, X, Y, Zₙ, R¹, R², R⁴, R⁵, R⁶ and R⁷
have the meanings given in Claim 1.

3. Process for the preparation of the 1-H-3-phenyl-5-cycloalkylpyrrolidine-2,4-diones according to Claim 1, **characterized in that**
(B) in the case of compounds of the formula (Ib) 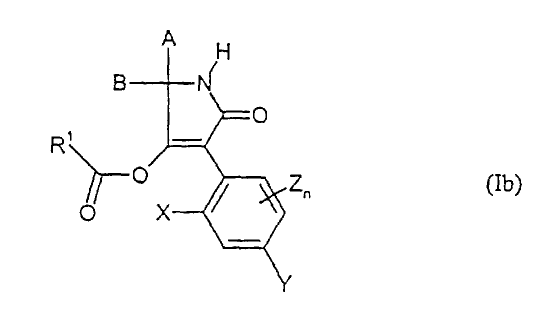 in which
A, B, X, Y, Z, R¹ and n have the meaning given in Claim 1.
compounds of the formula (Ia) 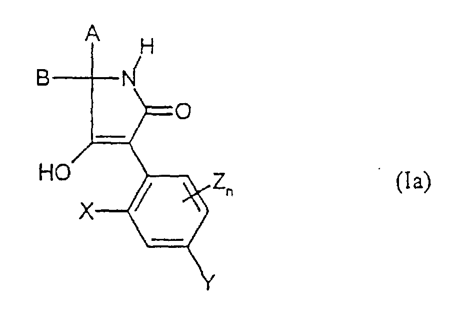 in which
A, B, X, Y, Z and n have the abovementioned meaning,
are reacted
a) with acid halides of the general formula (III) 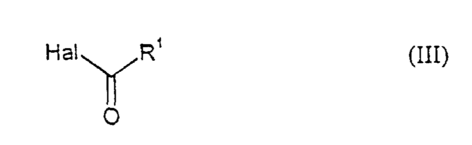 in which
R1 has the meaning given in Claim 1 and
Hal represents halogen, in particular chlor-ine and bromine,
if appropriate in the presence of a diluent and if appropriate in the presence of an acid-binding agent,
or
β) with carboxylic anhydrides of the general formula (IV)
R¹-CO-O-CO-R¹ (IV)
in which
R¹ has the meaning given in Claim 1,
if appropriate in the presence of a diluent and if appropriate in the presence of an acid-binding agent;
or
(C) in the case of compounds of the formula (Ic-1) 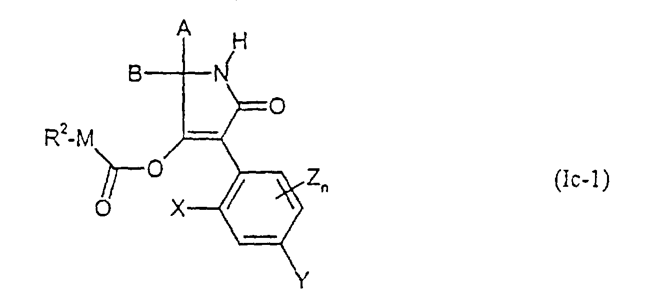 in which
A, B, X, Y, Z, R² and n have the meaning given in Claim 1
and
M represents oxygen or sulphur,
compounds of the formula (Ia) 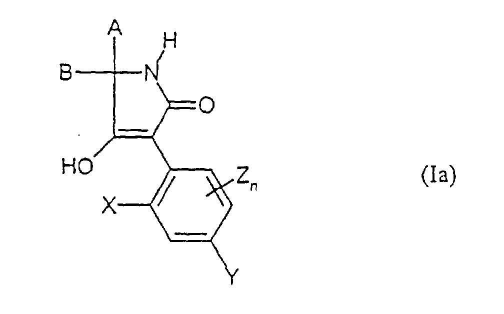 in which
A, B, X, Y, Z and n have the meaning given in Claim 1,
are reacted with chloroformic ester or chloroformic thioester of the general formula (V)
R²-M-CO-Cl (V)
in which
R² and M have the meaning given in Claim 1,
if appropriate in the presence of a diluent and if appropriate in the presence of an acid-binding agent;
or
(D) in the case of compounds of the formula (Ic-2) 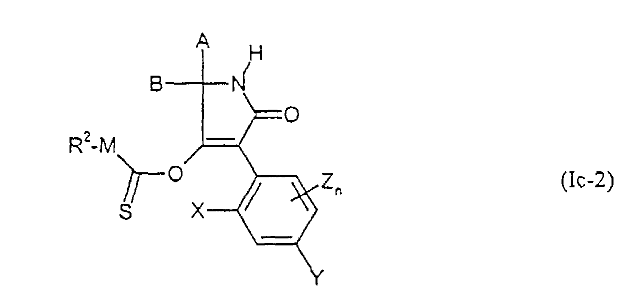 in which
A, B, R², X, Y, Z and n have the meaning given in Claim 1
and
M represents oxygen or sulphur,
compounds of the formula (Ia) 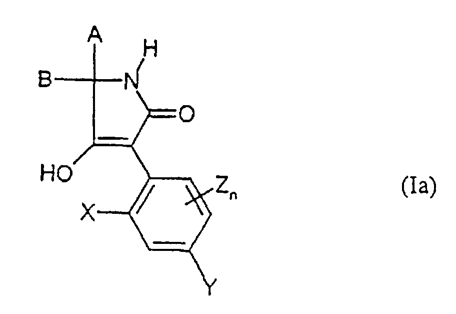 in which
A, B, X, Y, Z and n have the meaning given in Claim 1,
are reacted
(a) with chloromonothioformic esters or chlorodithioformic esters of the general formula (VI) 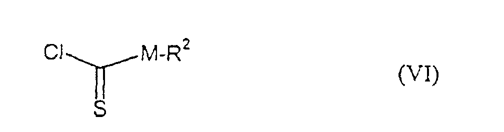 in which
M and R² have the meaning given in Claim 1,
if appropriate in the presence of a diluent and if appropriate in the presence of an acid-binding agent.
or
β) with carbon disulphide and subsequently with alkyl halides of the general formula (VII)
R²-Hal (VII)
in which
R² has the meaning given in Claim 1
and
Hal represents chlorine, bromine or iodine,
in appropriate in the presence of a diluent;
or
(F) in the case of compounds of the formula (Ie) 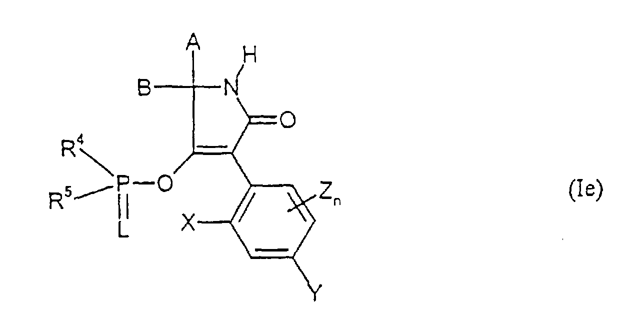 in which
A, B, L, X, Y, Z, R⁴, R⁵ and n have the meaning given in Claim 1,
1-H-3-phenyl-5-cycloalkylpyrrolidine-2,4-diones of the formula (Ia) or their enols 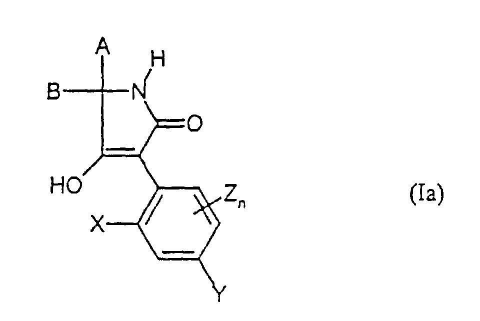 in which
A, B, X, Y, Z and n have the meaning given in Claim 1,
are reacted with phosphorus compounds of the general formula (IX) 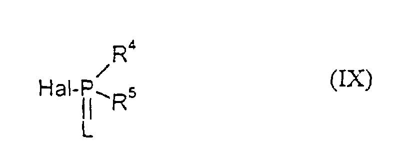 in which
L, R⁴ and R⁵ have the meaning given in Claim 1
and
Hal represents halogen, in particular chlorine and bromine,
if appropriate in the presence of a diluent and if appropriate in the presence of an acid-binding agent;
or
(H) in the case of compounds of the formula (Ig) 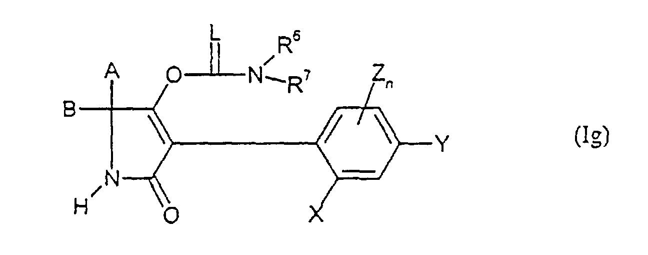 in which
A, B, L, X, Y, Z, R⁶, R⁷ and n have the meaning given in Claim 1,
compounds of the formula (Ia) 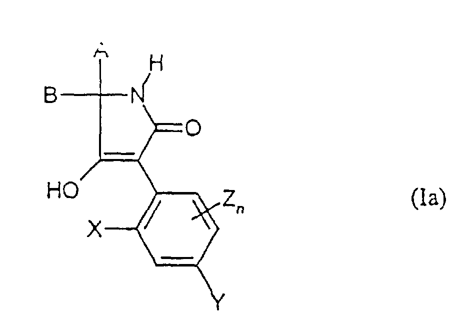 in which
A, B, X, Y, Z and n have the meaning given in Claim 1,
are reacted
a) with compounds of the general formula (XII)
R⁶-N=C=L (XII)
in which
L and R⁶ have the meaning given in Claim 1,
if appropriate in the presence of a diluent and if appropriate in the presence of a catalyst
or
b) with carbamoyl chlorides or thiocarbamoyl chlorides of the general formula (XIII) 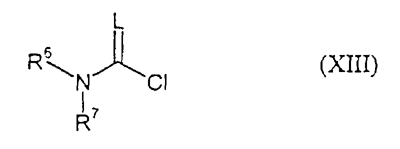 in which
L, R⁶ and R⁷ have the meaning given in Claim 1,
if appropriate in the presence of a diluent and if appropriate in the presence of an acid-binding agent.

4. Pesticides and herbicides, **characterized in that** they contain at least one 1-H-3-phenyl-5-cycloalkylpyrrolidine-2,4-dione of the formula (I) according to Claim 1.

5. Use of 1-H-3-phenyl-5-cycloalkylpyrrolidine-2,4-dione of the formula (I) according to Claim 1 for combating pests and undesired vegetation.

6. Method of combating pests, **characterized in that** 1-H-3-phenyl-5-cycloalkylpyrrolidine-2,4-diones of the formula (I) according to Claim 1 are allowed to act on pests, undesired plants and/or their environment.

7. Process for the preparation of pesticides, **characterized in that** 1-H-3-phenyl-5-cycloalkylpyrrolidine-2,4-diones of the formula (I) according to Claim 1 are mixed with extenders and/or surface-active agents.

## Revendications

1. 1-H-3-phényl-5-cycloalkylpyrrolidone-2,4-diones de formule (I) 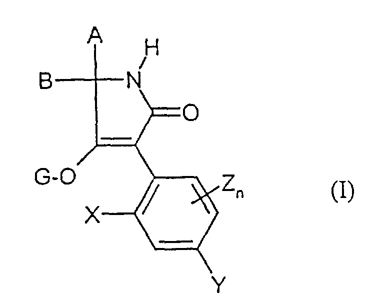 dans laquelle
A représente un groupe cycloalkyle en C₃ à C₆,
B représente un groupe méthyle, éthyle, propyle, isopropyle, butyle, isobutyle, s-butyle ou tertiobutyle,
x représente un groupe méthyle, éthyle, propyle, isopropyle, du fluor, du chlore ou du brome,
Y représente un groupe méthyle, éthyle, propyle, isopropyle, butyle, isobutyle, tertio-butyle, du fluor, du chlore ou du brome,
z représente un groupe méthyle, éthyle, propyle, isopropyle, butyle, isobutyle ou tertio-butyle,
n a la valeur 0 ou 1,
G représente les groupes 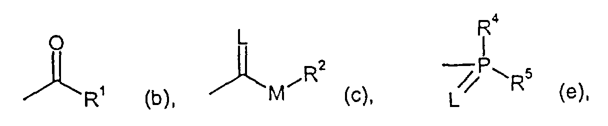 ou 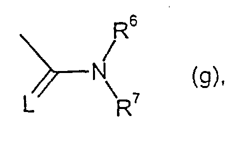 dans lesquels
L et M représentent de l'oxygène et/ou du soufre,
R¹ est un groupe alkyle en C₁ à C₁₄,
R² est un groupe alkyle en C₁ à C₁₄,
R₄ et R⁵ représentent, indépendamment l'un de l'autre, un groupe alkyle en C₁ à C₄ ou un groupe alkylthio en C₁ à C₄,
R⁶ et R⁷ représentent, indépendamment l'un de l'autre, un groupe alkyle en C₁ à C₄ ou forment conjointement avec l'atome d'azote auquel ils sont liés un noyau alkylénique en C₄ à C₆ éventuellement interrompu par de l'oxygène ou du soufre.

2. 1-H-3-phényl-5-cycloalkylpyrrolidine-2,4-diones suivant la revendication 1, répondant aux formules suivantes 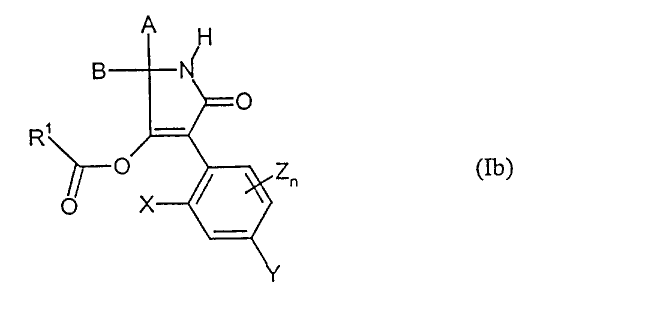 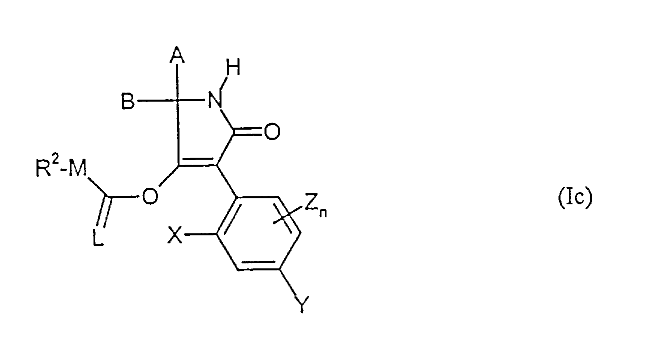 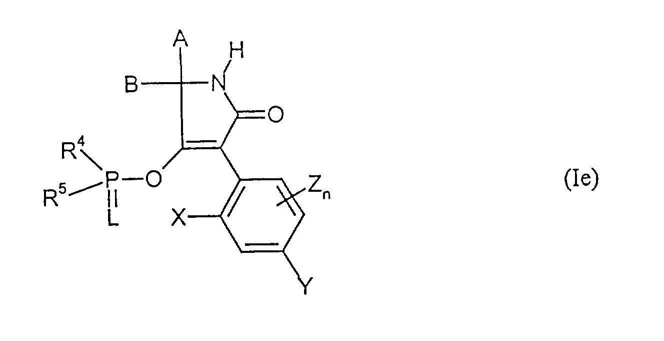 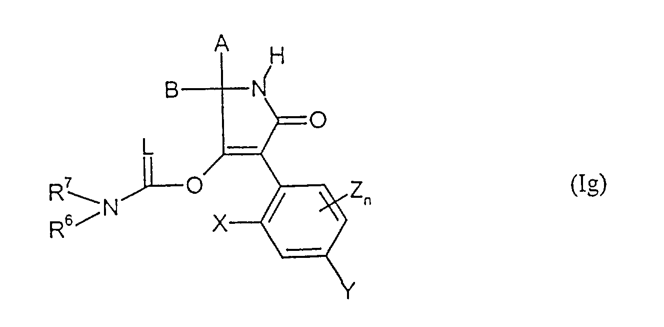 dans lesquelles
A, B, L, M, X, Y, Zₙ, R¹, R², R⁴, R⁵, R⁶ et R⁷ ont les définitions indiquées dans la revendication 1.

3. Procédé de production de 1-H-3-phényl-5-cycloalkylpyrrolidine-2,4-diones suivant la revendication 1, **caractérisé en ce que**
(B) dans le cas de composés de formule (Ib) 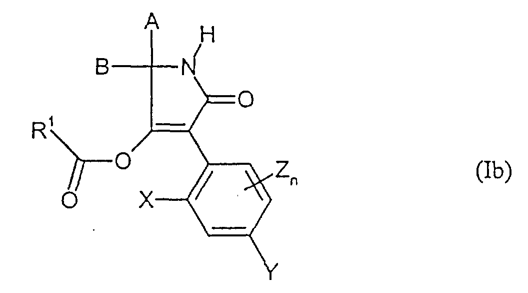 dans laquelle
A, B, X, Y, Z, R¹ et n ont la définition indiquée dans la revendicaiton 1,
on fait réagir des composés de formule (Ia) 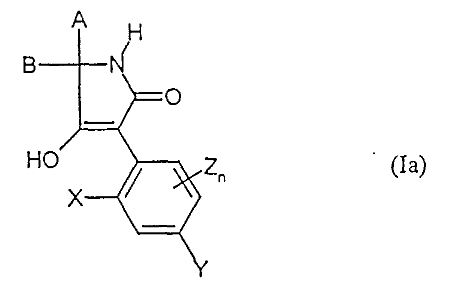 dans laquelle
A, B, X, Y, Z et n ont la définition indiquée ci-dessus,
a) avec des halogénures d'acides de formule générale (III) 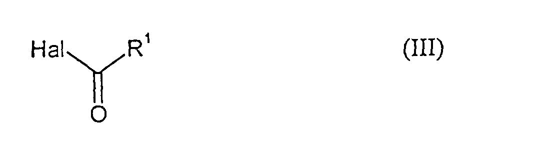 dans laquelle
R¹ a la définition indiquée dans la revendication 1 et
Hal représente un halogène, en particulier le chlore ou le brome,
éventuellement en présence d'un diluant et, le cas échéant, en présence d'un accepteur d'acide
ou bien
β) avec des anhydrides d'acides carboxyliques de formule générale (IV)
R¹-CO-O-CO-R¹ (IV)
dans laquelle
R¹ a la définition indiquée ci-dessus,
le cas échéant en présence d'un diluant et en présence éventuelle d'un accepteur d'acide ;
ou bien
(C) dans le cas de composés de formule (Ic-1) 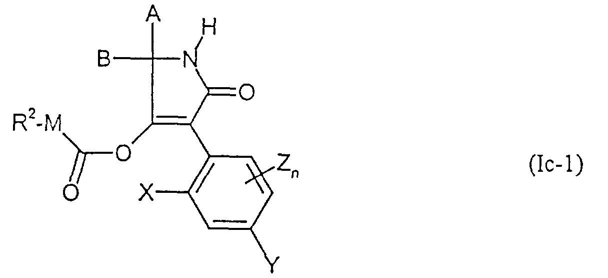 dans laquelle
A, B, X, Y, Z, R² et n ont la définition indiquée dans la revendication 1,
et
M représente l'oxygène ou le soufre,
on fait réagir des composés de formule (Ia) 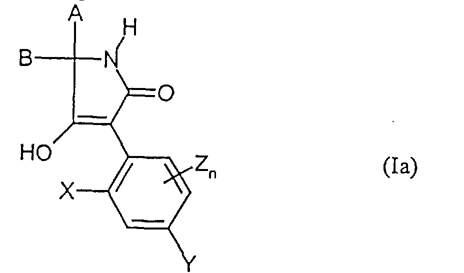 dans laquelle
A, B, X, Y, Z et n ont la définition indiquée dans la revendication 1,
avec des esters d'acide chloroformique ou des thioesters d'acide chloroformique de formule générale (V)
R²-M-CO-Cl (V)
dans laquelle
R² et M ont la définition indiquée dans la revendication 1,
éventuellement en présence d'un diluant et, le cas échéant, en présence d'un accepteur d'acide ;
ou bien
(D) dans le cas de composés de formule (Ic-2) 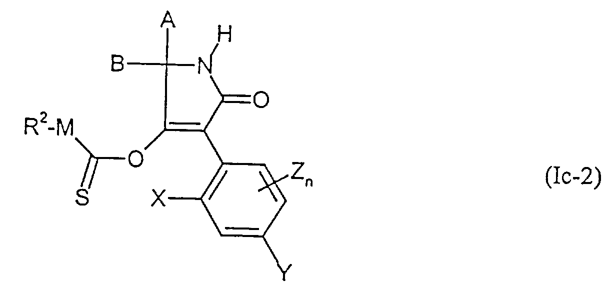 dans laquelle
A, B, R², X, Y, Z et n ont la définition indiquée dans la revendication 1,
et
M représente l'oxygène ou le soufre,
on fait réagir des composés de formule (Ia) 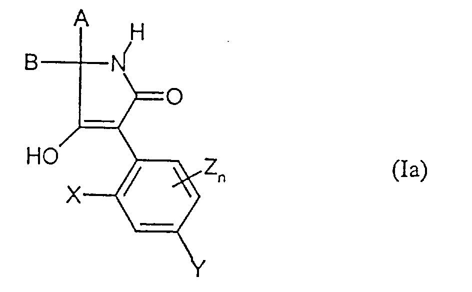 dans laquelle
A, B, X, Y, Z et n ont la définition indiquée dans la revendication 1,
(a) avec des esters d'acide monochlorothioformique ou des esters d'acide chlorodithioformique de formule générale (VI) 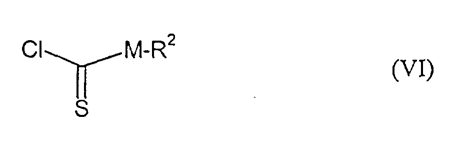 dans laquelle
M et R² ont la définition indiquée dans la revendication 1,
le cas échéant en présence d'un diluant et en présence éventuelle d'un accepteur d'acide,
ou bien
β) avec le sulfure de carbone, puis avec des halogénures d'alkyle de formule générale (VII)
R²-Hal (VII)
dans laquelle
R² a la définition indiquée dans la revendication 1
et
Hal représente le chlore, le brome ou l'iode,
éventuellement en présence d'un diluant ;
ou bien
(F) dans le cas de composés de formule (Ie) 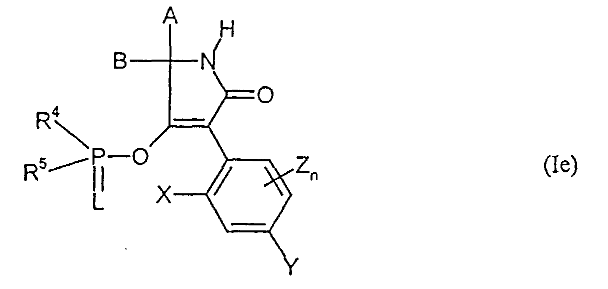 dans laquelle
A, B, L, X, Y, Z, R⁴, R⁵ et n ont la définition indiquée dans la revendication 1,
on fait réagir des 1-H-3-phényl-5-cycloalkylpyrrolidine-2,4-diones de formule (Ia) ou leurs énols 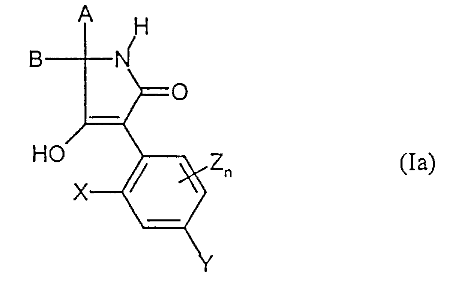 formule dans laquelle
A, B, X, Y, Z et n ont la définition indiquée dans la revendication 1,
avec des composés de phosphore de formule générale (IX) 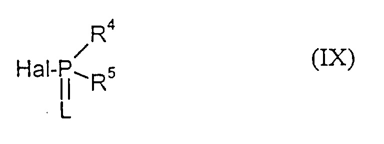 dans laquelle
L, R⁴ et R⁵ ont la définition indiquée dans la revendication 1,
et
Hal représente un halogène, en particulier le chlore ou le brome,
le cas échéant en présence d'un diluant et en présence éventuelle d'un accepteur d'acide ;
ou bien
(H) dans le cas de composés de formule (Ig) 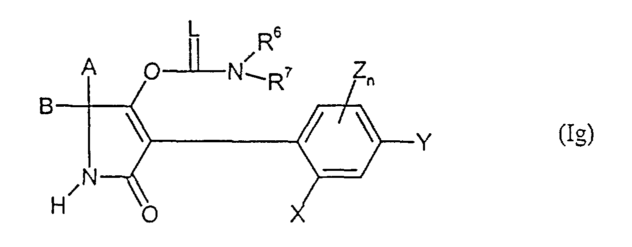 dans laquelle
A, B, L, X, Y, Z, R⁶, R⁷ et n ont la définition indiquée dans la revendication 1,
on fait réagir des composés de formule (Ia) 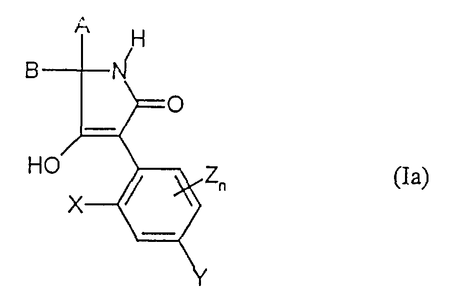 dans laquelle
A, B, X, Y, Z et n ont la définition indiquée dans la revendication 1,
a) avec des composés de formule générale (XII)
R⁶-N=C=L (XII)
dans laquelle
L et R⁶ ont la définition indiquée dans la revendication 1,
le cas échéant en présence d'un diluant et en présence éventuelle d'un catalyseur
ou bien
b) avec des chlorures d'acide carbamique ou des chlorures d'acide thiocarbamique de formule générale (XIII) 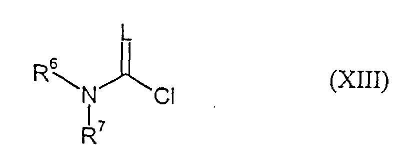 dans laquelle
L, R⁶ et R⁷ ont la définition indiquée dans la revendication 1
le cas échéant en présence d'un diluant et en présence éventuelle d'un accepteur d'acide.
4. Pesticides et herbicides, **caractérisés par** une teneur en au moins une 1-H-3-phényl-5-cycloalkylpyrrolidine-2,4-dione de formule (I) suivant la revendication 1.
5. Utilisation de 1-H-3-phényl-5-cycloalkylpyrrolidine-2,4-diones de formule (I) suivant la revendication 1, pour combattre des parasites et une végétation non désirée.
6. Procédé pour combattre des parasites, **caractérisé en ce qu'**on fait agir des 1-H-3-phényl-5-cycloalkylpyrrolidine-2,4-diones de formule (I) suivant la revendication 1 sur les parasites, sur des plantes indésirables et/ou sur leur milieu.
7. Procédé de préparation de compositions pesticides, **caractérisé en ce qu'**on mélange des 1-H-3-phényl-5-cycloalkylpyrrolidine-2,4-diones de formule (I) suivant la revendication 1 avec des diluants et/ou des agents tensioactifs.
